# EUROPEAN PATENT SPECIFICATION

(11) **EP 3 179 538 B1**
(45) Date of publication and mention of the grant of the patent: **11.12.2019**
(21) Application number: 17151795.6
(22) Date of filing: 10.10.2013
(51) Int. Cl.: H01M 4/06, H01M 4/62, H01M 6/40, H01M 2/14, H01M 2/16, H01M 6/16, H01G 11/30, H01G 11/36

(54) **PRINTED ENERGY STORAGE DEVICE**
GEDRUCKTE ENERGIESPEICHERVORRICHTUNG
DISPOSITIF IMPRIMÉ DE STOCKAGE D'ÉNERGIE

(30) Priority: 10.10.2012 US 201261712219 P
(43) Date of publication of application: 14.06.2017
(62) Divisional of application: 13846066.2
(73) Proprietor: Printed Energy Pty Ltd, Brisbane, QLD 4000 (AU)
(72) Inventor: Lockett, Vera N., Phoenix, Arizona 85044 (US); Gustafson, John G., Chandler, Arizona 85286 (US); Hartman, Alexandra E., Tolleson, Arizona 85353 (US); Lowenthal, Mark D., Gilbert, Arizona 85297 (US); Ray, William J., Fountain Hills, Arizona 85268 (US)
(74) Representative: Barker Brettell LLP

(56) References cited:
- WO-A2-2012/037171
- US-A1- 2009 246 625
- US-A1- 2011 045 337

## Description

### CROSS REFERENCE TO RELATED APPLICATIONS

This application claims priority benefit of U.S. Provisional Patent Application Serial No. 61/712,219, filed October 10, 2012, entitled "PRINTED ENERGY STORAGE DEVICE".

### BACKGROUND

### Field

This invention relates to printed electrodes for printed energy storage devices and to printed energy storage devices.

### Description of the Related Art

Thin and flexible energy storage devices are needed for powering thin and/or small electronic devices in the consumer market. For example, lights and sound in greeting cards, thin advertisement tools like magazine inserts, skin care products, safe pass cards, some miniature medical devices, etc. can be powered by using thin batteries. Some thin batteries already exist on the market (e.g., zinc carbon batteries produced by Enfucell Oy Ltd. of Vantaa, Finland and Blue Spark Technologies, Inc. of Westlake, Ohio, and lithium polymer batteries produced by Solicore, Inc. of Lakeland, Florida). These batteries generally have a thickness from about 0.45-0.7 mm. They are sealed in a pouch unit cells with two poles for wired connection to devices which need power.

Zinc-manganese dioxide (Zn/MnO₂) batteries are primary batteries (e.g., one time use). These batteries can be filled with an aqueous solution of zinc and ammonia salts or potassium hydroxide. They have an initial voltage of 1.5-1.6V and are designed for low or moderate current drain. Shelf life of these batteries is 1-3 years. Main advantages of Zn/MnO₂ batteries are cost and safety. They are the most affordable batteries on the market due to cheap and abundant raw materials for the battery build. These materials are considered "green" due to non-toxicity.

US 2011/045337 (A1) discloses a vacuum-sealing-type flexible-film primary battery and a method of manufacturing the same. The primary battery includes a battery assembly comprising a positive electrode plate including a positive electrode collector having a first conductive carbon layer disposed on a surface-treated inner surface of a first pouch and a positive electrode layer disposed on the first conductive carbon layer of the positive electrode collector, a negative electrode plate including a negative electrode collector having a second conductive carbon layer disposed on a surface-treated inner surface of a second pouch and a negative electrode layer disposed on the second conductive carbon layer of the negative electrode collector, and an adhesion/post-injection polymer electrolyte layer interposed between the positive electrode plate and the negative electrode plate, wherein the battery assembly is completely sealed. The flexible-film primary battery may employ the pouch as a collector film. Also, the flexible-film primary battery may be completely sealed using the pouch t. Furthermore, the flexible-film primary battery may be manufactured using a screen printing technique.

WO 2012/037171 (A2) discloses an electrochemical cell which includes a solid-state, printable anode layer, cathode layer and non-aqueous gel electrolyte layer coupled to the anode layer and cathode layer. The electrolyte layer provides physical separation between the anode layer and the cathode layer, and comprises a composition configured to provide ionic communication between the anode layer and cathode layer by facilitating transmission of multivalent ions between the anode layer and the cathode layer.

US 2009/246625 (A1) discloses lithium-ion batteries based on aligned graphene ribbon anodes, V₂O₅ graphene ribbon composite cathodes, and ionic liquid electrolytes. The lithium-ion batteries have excellent performance metrics of cell voltages, energy densities, and power densities.

### SUMMARY

In accordance with an aspect of the invention, we provide a printed electrode in accordance with claim 1. Optional features are recited in the dependent claims.

The second electrode may have a homogeneous paste comprising the carbon nanotubes and the ionic liquid. The second electrode may have manganese dioxide. In some embodiments, the second electrode can include a conductive carbon. The conductive carbon can include graphite.

In some embodiments, the current collector can have at least one of nickel flakes, graphene flakes, and graphite powder. The current collector may have nickel flakes. The current collector may have graphene flakes. The current collector may have nickel flakes and graphene flakes. The current collector may have nickel flakes, graphene flakes, and graphite powder. The current collector may have nickel flakes and carbon nanotubes. The current collector may have nickel flakes, graphene flakes, and carbon nanotubes. The current collector may have nickel flakes, graphene flakes, carbon nanotubes, and graphite powder. The current collector may have nickel flakes, carbon nanotubes, and graphite powder. In some embodiments, the current collector can have polyvinylidene difluoride.

In some embodiments, the separator can have microspheres. The microspheres may include at least one of glass, alumina, silica, polystyrene, and melamine. The microspheres may be hollow. The microspheres may be solid. In some embodiments, the microspheres can have a diameter from about 0.5 microns to about 30 microns. In some embodiments, the separator can include polyvinylidene difluoride.

A layer of a printed energy storage device, for example a printed zinc manganese-dioxide (Zn/MnO₂) battery, includes a salt having an anion, and an ionic liquid including the anion.

The ionic liquid includes a cation selected from the group consisting of 1-ethyl-3-methylimidazolium, butyltrimethylammonium, 1-butyl-3-methylimidazolium, 1-methyl-3-propylimidazolium, 1-hexyl-3-methylimidazolium, choline, ethylammonium, tributylmethylphosphonium, tributyl(tetradecyl)phosphonium, trihexyl(tetradecyl)phosphonium, 1-ethyl-2,3-methylimidazolium, 1-butyl-1-methylpiperidinium, diethylmethylsulfonium, 1-methyl-3-propylimidazolium, 1-methyl-1-propylpiperidinium, 1-butyl-2-methylpyridinium, 1-butyl-4-methylpyridinium, 1-butyl-1-methylpyrrolidinium, and diethylmethylsulfonium.

The anion can be selected from the group consisting of tetrafluoroborate, tris(pentafluoroethyl)trifluorophosphate, trifluoromethanesulfonate, hexafluorophosphate, tetrafluoroborate, ethyl sulfate, dimethyl phosphate, methansulfonate, triflate, tricyanomethanide, dibutylphosphate, bis(trifluoromethylsulfonyl)imide, bis-2,4,4-(trimethylpentyl) phosphinate, iodide, chloride, bromide, and nitrate.

In some embodiments, the ionic liquid can include 1-ethyl-3-methylimidazolium tetrafluoroborate (C2mimBF4). The salt includes a zinc salt. The salt may include zinc tetrafluoroborate. In some embodiments, the salt can include zinc chloride. In some embodiments, the salt can include zinc bis(trifluoromethanesulfonyl)imide. In some embodiments, the salt can include zinc sulfate. In some embodiments, the salt can include zinc nitrate. In some embodiments, the salt can include zinc carbonate.

The printed energy storage device may have a first electrode, a second electrode, and a separator between the first electrode and the second electrode. In some embodiments, the printed energy storage device can include an intermediate layer. The intermediate layer may be between the first electrode and the separator. The intermediate layer may be between the second electrode and the separator. In some embodiments, the printed energy storage device can include a current collector electrically coupled to the first electrode or the second electrode.

The layer may be the first electrode.

In some embodiments, at least one of the first electrode, the second electrode, the separator, the intermediate layer, and the current collector can have a polymer selected from the group consisting of polyvinyl pyrrolidone (PVP), polyvinyl alcohol (PVA), polyvinylidene fluoride, polyvinylidene fluoride-trifluoroethylene, polytetrafluoroethylene, polydimethylsiloxane, polyethelene, polypropylene, polyethylene oxide, polypropylene oxide, polyethylene glycolhexafluoropropylene, polyethylene terefphtalatpolyacrylonitryle, polyvinyl butyral, polyvinylcaprolactam, polyvinyl chloride; polyimide polymers and copolymers (including aliphatic, aromatic and semi-aromatic polyimides), polyamides, polyacrylamide, acrylate and (meth)acrylate polymers and copolymers such as polymethylmethacrylate, polyacrylonitrile, acrylonitrile butadiene styrene, allylmethacrylate, polystyrene, polybutadiene, polybutylene terephthalate, polycarbonate, polychloroprene, polyethersulfone, nylon, styrene-acrylonitrile resin; polyethylene glycols, clays such as hectorite clays, garamite clays, organomodified clays; saccharides and polysaccharides such as guar gum, xanthan gum, starch, butyl rubber, agarose, pectin; celluloses and modified celluloses such as hydroxyl methylcellulose, methylcellulose, ethyl cellulose, propyl methylcellulose, methoxy cellulose, methoxy methylcellulose, methoxy propyl methylcellulose, hydroxy propyl methylcellulose, carboxy methylcellulose, hydroxy ethylcellulose, ethyl hydroxyl ethylcellulose, cellulose ether, cellulose ethyl ether, and chitosan.

In some embodiments, at least one of the first electrode, the second electrode, the separator, and the current collector can have polyvinylidene difluoride.

In some embodiments, at least one of the second electrode and the current collector can have carbon nanotubes. The carbon nanotubes may include single-wall carbon nanotubes. The carbon nanotubes may include multi-wall carbon nanotubes.

The second electrode may have a mixture including the carbon nanotubes and the ionic liquid. The second electrode may have manganese dioxide. In some embodiments, the second electrode can include a conductive carbon. The conductive carbon can include graphite.

In some embodiments, the current collector can have at least one of nickel flakes, graphene flakes, and graphite powder. The current collector may have nickel flakes. The current collector may have graphene flakes. The current collector may have nickel flakes and graphene flakes. The current collector may have nickel flakes, graphene flakes, and graphite powder. The current collector may have nickel flakes and carbon nanotubes. The current collector may have nickel flakes, graphene flakes, and carbon nanotubes. The current collector may have nickel flakes, graphene flakes, carbon nanotubes, and graphite powder. The current collector may have nickel flakes, carbon nanotubes, and graphite powder.

In some embodiments, the separator can have microspheres. The microspheres may include at least one of glass, alumina, silica, polystyrene, and melamine. The microspheres may be hollow. The microspheres may be solid. In some embodiments, the microspheres can have a diameter from about 0.5 microns to about 30 microns.

In some embodiments, the intermediate layer can include polyvinyl alcohol.

A layer of a printed energy storage device, for example a printed zinc manganese-dioxide (Zn/MnO₂) battery, may have a salt including zinc tetrafluoroborate, and an ionic liquid having 1-ethyl-3-methylimidazolium tetrafluoroborate.

The printed energy storage device may include a first electrode, a second electrode, and a separator between the first electrode and the second electrode. In some embodiments, the printed energy storage device can include an intermediate layer. The intermediate layer may be between the first electrode and the separator. The intermediate layer may be between the second electrode and the separator. In some embodiments, the printed energy storage device can include a current collector electrically coupled to the first electrode or the second electrode.

The layer may be the first electrode.

In some embodiments, at least one of the first electrode, the second electrode, the separator, the intermediate layer, and the current collector can have a polymer selected from the group consisting of polyvinyl pyrrolidone (PVP), polyvinyl alcohol (PVA), polyvinylidene fluoride, polyvinylidene fluoride-trifluoroethylene, polytetrafluoroethylene, polydimethylsiloxane, polyethelene, polypropylene, polyethylene oxide, polypropylene oxide, polyethylene glycolhexafluoropropylene, polyethylene terefphtalatpolyacrylonitryle, polyvinyl butyral, polyvinylcaprolactam, polyvinyl chloride; polyimide polymers and copolymers (including aliphatic, aromatic and semi-aromatic polyimides), polyamides, polyacrylamide, acrylate and (meth)acrylate polymers and copolymers such as polymethylmethacrylate, polyacrylonitrile, acrylonitrile butadiene styrene, allylmethacrylate, polystyrene, polybutadiene, polybutylene terephthalate, polycarbonate, polychloroprene, polyethersulfone, nylon, styrene-acrylonitrile resin; polyethylene glycols, clays such as hectorite clays, garamite clays, organomodified clays; saccharides and polysaccharides such as guar gum, xanthan gum, starch, butyl rubber, agarose, pectin; celluloses and modified celluloses such as hydroxyl methylcellulose, methylcellulose, ethyl cellulose, propyl methylcellulose, methoxy cellulose, methoxy methylcellulose, methoxy propyl methylcellulose, hydroxy propyl methylcellulose, carboxy methylcellulose, hydroxy ethylcellulose, ethyl hydroxyl ethylcellulose, cellulose ether, cellulose ethyl ether, and chitosan.

In some embodiments, at least one of the first electrode, the second electrode, the separator, and the current collector can have polyvinylidene difluoride.

In some embodiments, at least one of the second electrode and the current collector can have carbon nanotubes. The carbon nanotubes may include single-wall carbon nanotubes. The carbon nanotubes may include multi-wall carbon nanotubes.

The second electrode may have a mixture including the carbon nanotubes and the ionic liquid. The second electrode may have manganese dioxide. In some embodiments, the second electrode can include a conductive carbon. The conductive carbon can include graphite.

In some embodiments, the current collector can have at least one of nickel flakes, graphene flakes, and graphite powder. The current collector may have nickel flakes. The current collector may have graphene flakes. The current collector may have nickel flakes and graphene flakes. The current collector may have nickel flakes, graphene flakes, and graphite powder. The current collector may have nickel flakes and carbon nanotubes. The current collector may have nickel flakes, graphene flakes, and carbon nanotubes. The current collector may have nickel flakes, graphene flakes, carbon nanotubes, and graphite powder. The current collector may have nickel flakes, carbon nanotubes, and graphite powder.

In some embodiments, the separator can have microspheres. The microspheres may include at least one of glass, alumina, silica, polystyrene, and melamine. The microspheres may be hollow. The microspheres may be solid. In some embodiments, the microspheres can have a diameter from about 0.5 microns to about 30 microns.

In some embodiments, the intermediate layer can include polyvinyl alcohol.

A planarization adhesion layer of a printed energy storage device, for example a printed zinc manganese-dioxide (Zn/MnO₂) battery, can include polyvinyl alcohol, a salt, an ionic liquid, where the ionic liquid includes a cation selected from the group consisting of 1-ethyl-3-methylimidazolium, butyltrimethylammonium, 1-butyl-3-methylimidazolium, 1-methyl-3-propylimidazolium, 1-hexyl-3-methylimidazolium, choline, ethylammonium, tributylmethylphosphonium, tributyl(tetradecyl)phosphonium, trihexyl(tetradecyl)phosphonium, 1-ethyl-2,3-methylimidazolium, 1-butyl-1-methylpiperidinium, diethylmethylsulfonium, 1-methyl-3-propylimidazolium, 1-methyl-1-propylpiperidinium, 1-butyl-2-methylpyridinium, 1-butyl-4-methylpyridinium, 1-butyl-1-methylpyrrolidinium, and diethylmethylsulfonium, and where the ionic liquid includes an anion selected from the group consisting of tetrafluoroborate, tris(pentafluoroethyl)trifluorophosphate, trifluoromethanesulfonate, hexafluorophosphate, tetrafluoroborate, ethyl sulfate, dimethyl phosphate, methansulfonate, triflate, tricyanomethanide, dibutylphosphate, bis(trifluoromethylsulfonyl)imide, bis-2,4,4-(trimethylpentyl) phosphinate, iodide, chloride, bromide, and nitrate.

The salt includes an anion that is the same as the anion of the ionic liquid. The salt includes a zinc salt. The salt may include zinc tetrafluoroborate and the ionic liquid can include 1-ethyl-3-methylimidazolium tetrafluoroborate. In some embodiments, the salt can include zinc chloride. In some embodiments, the salt can include zinc bis(trifluoromethanesulfonyl)imide. In some embodiments, the salt can include zinc sulfate. In some embodiments, the salt can include zinc nitrate. In some embodiments, the salt can include zinc carbonate.

The printed energy storage device may have a first electrode, a second electrode, and a separator between the first electrode and the second electrode.

The planarization adhesion layer may be between the first electrode and the separator. The planarization adhesion layer may be between the second electrode and the separator. In some embodiments, the printed energy storage device can include a current collector electrically coupled to the first electrode or the second electrode. In some embodiments, the printed energy storage device can include a current collector electrically coupled to the first electrode or the second electrode.

In some embodiments, at least one of the first electrode, the second electrode, the separator, the planarization adhesion layer, and the current collector can have a polymer selected from the group consisting of polyvinyl pyrrolidone (PVP), polyvinyl alcohol (PVA), polyvinylidene fluoride, polyvinylidene fluoride-trifluoroethylene, polytetrafluoroethylene, polydimethylsiloxane, polyethelene, polypropylene, polyethylene oxide, polypropylene oxide, polyethylene glycolhexafluoropropylene, polyethylene terefphtalatpolyacrylonitryle, polyvinyl butyral, polyvinylcaprolactam, polyvinyl chloride; polyimide polymers and copolymers (including aliphatic, aromatic and semi-aromatic polyimides), polyamides, polyacrylamide, acrylate and (meth)acrylate polymers and copolymers such as polymethylmethacrylate, polyacrylonitrile, acrylonitrile butadiene styrene, allylmethacrylate, polystyrene, polybutadiene, polybutylene terephthalate, polycarbonate, polychloroprene, polyethersulfone, nylon, styrene-acrylonitrile resin; polyethylene glycols, clays such as hectorite clays, garamite clays, organomodified clays; saccharides and polysaccharides such as guar gum, xanthan gum, starch, butyl rubber, agarose, pectin; celluloses and modified celluloses such as hydroxyl methylcellulose, methylcellulose, ethyl cellulose, propyl methylcellulose, methoxy cellulose, methoxy methylcellulose, methoxy propyl methylcellulose, hydroxy propyl methylcellulose, carboxy methylcellulose, hydroxy ethylcellulose, ethyl hydroxyl ethylcellulose, cellulose ether, cellulose ethyl ether, and chitosan.

In some embodiments, at least one of the first electrode, the second electrode, the separator, and the current collector can have polyvinylidene difluoride.

In some embodiments, at least one of the second electrode and the current collector can have carbon nanotubes. The carbon nanotubes may include single-wall carbon nanotubes. The carbon nanotubes may include multi-wall carbon nanotubes.

The second electrode may have a mixture including the carbon nanotubes and the ionic liquid. The second electrode may have manganese dioxide. In some embodiments, the second electrode can include a conductive carbon. The conductive carbon can include graphite.

In some embodiments, the current collector can have at least one of nickel flakes, graphene flakes, and graphite powder. The current collector may have nickel flakes, for example a current collector electrically coupled to the first electrode. The current collector may have graphene flakes, for example a current collector electrically coupled to the second electrode. The current collector may have nickel flakes and graphene flakes. The current collector may have nickel flakes, graphene flakes, and graphite powder. The current collector may have nickel flakes and carbon nanotubes. The current collector may have nickel flakes, graphene flakes, and carbon nanotubes. The current collector may have nickel flakes, graphene flakes, carbon nanotubes, and graphite powder. The current collector may have nickel flakes, carbon nanotubes, and graphite powder.

In some embodiments, the separator can have microspheres. The microspheres may include at least one of glass, alumina, silica, polystyrene, and melamine. The microspheres may be hollow. The microspheres may be solid. In some embodiments, the microspheres can have a diameter from about 0.5 microns to about 30 microns.

At least one of the first electrode and the second electrode includes the ionic liquid. The separator may include the ionic liquid.

An electrode of a printed energy storage device, for example a printed zinc manganese-dioxide (Zn/MnO₂) battery, can include carbon nanotubes, and an ionic liquid, where the ionic liquid includes a cation selected from the group consisting of 1-ethyl-3-methylimidazolium, butyltrimethylammonium, 1-butyl-3-methylimidazolium, 1-methyl-3-propylimidazolium, 1-hexyl-3-methylimidazolium, choline, ethylammonium, tributylmethylphosphonium, tributyl(tetradecyl)phosphonium, trihexyl(tetradecyl)phosphonium, 1-ethyl-2,3-methylimidazolium, 1-butyl-1-methylpiperidinium, diethylmethylsulfonium, 1-methyl-3-propylimidazolium, 1-methyl-1-propylpiperidinium, 1-butyl-2-methylpyridinium, 1-butyl-4-methylpyridinium, 1-butyl-1-methylpyrrolidinium, and diethylmethylsulfonium, and where the ionic liquid includes an anion selected from the group consisting of tetrafluoroborate, tris(pentafluoroethyl)trifluorophosphate, trifluoromethanesulfonate, hexafluorophosphate, tetrafluoroborate, ethyl sulfate, dimethyl phosphate, methansulfonate, triflate, tricyanomethanide, dibutylphosphate, bis(trifluoromethylsulfonyl)imide, bis-2,4,4-(trimethylpentyl) phosphinate, iodide, chloride, bromide, and nitrate.

The carbon nanotubes may include single-wall carbon nanotubes. The carbon nanotubes may include multi-wall carbon nanotubes. The carbon nanotubes may be ground. In some embodiments, the carbon nanotubes and the ionic liquid can form a homogeneous mixture.

In some embodiments, the ionic liquid can include 1-ethyl-3-methylimidazolium tetrafluoroborate.

In some embodiments, the electrode can include manganese dioxide. In some embodiments, the electrode can include graphite powder.

The printed energy storage device may further include a second electrode and a separator between the electrode and the second electrode.

In some embodiments, the printed energy storage device can include an intermediate layer. The intermediate layer may be between the first electrode and the separator. The intermediate layer may be between the second electrode and the separator. In some embodiments, the printed energy storage device can include a current collector electrically coupled to the first electrode or the second electrode.

In some embodiments, at least one of the second electrode, the separator, and the intermediate layer can include the ionic liquid. In some embodiments, at least one of the second electrode, the separator, and the intermediate layer can include a salt. The salt includes an anion that is the same as an anion of the ionic liquid.

The salt includes a zinc salt. In some embodiments, the ionic liquid can include 1-ethyl-3-methylimidazolium tetrafluoroborate (C2mimBF4) and the salt can include zinc tetrafluorborate. In some embodiments, the salt can include zinc chloride. In some embodiments, the salt can include zinc bis(trifluoromethanesulfonyl)imide. In some embodiments, the salt can include zinc sulfate. In some embodiments, the salt can include zinc nitrate. In some embodiments, the salt can include zinc carbonate.

In some embodiments, at least one of the first electrode, the second electrode, the separator, the intermediate layer, and the current collector can have a polymer selected from the group consisting of polyvinyl pyrrolidone (PVP), polyvinyl alcohol (PVA), polyvinylidene fluoride, polyvinylidene fluoride-trifluoroethylene, polytetrafluoroethylene, polydimethylsiloxane, polyethelene, polypropylene, polyethylene oxide, polypropylene oxide, polyethylene glycolhexafluoropropylene, polyethylene terefphtalatpolyacrylonitryle, polyvinyl butyral, polyvinylcaprolactam, polyvinyl chloride; polyimide polymers and copolymers (including aliphatic, aromatic and semi-aromatic polyimides), polyamides, polyacrylamide, acrylate and (meth)acrylate polymers and copolymers such as polymethylmethacrylate, polyacrylonitrile, acrylonitrile butadiene styrene, allylmethacrylate, polystyrene, polybutadiene, polybutylene terephthalate, polycarbonate, polychloroprene, polyethersulfone, nylon, styrene-acrylonitrile resin; polyethylene glycols, clays such as hectorite clays, garamite clays, organomodified clays; saccharides and polysaccharides such as guar gum, xanthan gum, starch, butyl rubber, agarose, pectin; celluloses and modified celluloses such as hydroxyl methylcellulose, methylcellulose, ethyl cellulose, propyl methylcellulose, methoxy cellulose, methoxy methylcellulose, methoxy propyl methylcellulose, hydroxy propyl methylcellulose, carboxy methylcellulose, hydroxy ethylcellulose, ethyl hydroxyl ethylcellulose, cellulose ether, cellulose ethyl ether, and chitosan.

In some embodiments, at least one of the electrode, the second electrode, the separator, and the current collector can include polyvinylidene difluoride.

In some embodiments, the current collector can have at least one of nickel flakes, graphene flakes, and graphite powder. The current collector may have nickel flakes, for example a current collector coupled to the second electrode. The current collector may have graphene flakes, for example a current collector coupled to the first electrode. The current collector may have nickel flakes and graphene flakes. The current collector may have nickel flakes, graphene flakes, and graphite powder. The current collector may have nickel flakes and carbon nanotubes. The current collector may have nickel flakes, graphene flakes, and carbon nanotubes. The current collector may have nickel flakes, graphene flakes, carbon nanotubes, and graphite powder. The current collector may have nickel flakes, carbon nanotubes, and graphite powder.

In some embodiments, the separator can have microspheres. The microspheres may include at least one of glass, alumina, silica, polystyrene, and melamine. The microspheres may be hollow. The microspheres may be solid. In some embodiments, the microspheres can have a diameter from about 0.5 microns to about 30 microns.

In some embodiments, the intermediate layer can include polyvinyl alcohol.

A printed energy storage device, for example a printed zinc manganese-dioxide (Zn/MnO₂) battery, can include a first electrode having zinc, a second electrode having manganese dioxide, a separator between the first electrode and the second electrode, and a current collector electrically connected to the first electrode or the second electrode, the current collector including conductive flakes.

In some embodiments, the current collector can include carbon nanotubes. The carbon nanotubes may include single-wall carbon nanotubes. The carbon nanotubes may include multi-wall carbon nanotubes.

In some embodiments, the conductive flakes can include at least one of nickel flakes, graphene flakes, and graphite powder. The current collector may have nickel flakes, for example a current collector electrically coupled to the first electrode. The current collector may have graphene flakes, for example a current collector electrically coupled to the second electrode. The current collector may have nickel flakes and graphene flakes. The current collector may have nickel flakes, graphene flakes, and graphite powder. The current collector may have nickel flakes and carbon nanotubes. The current collector may have nickel flakes, graphene flakes, and carbon nanotubes. The current collector may have nickel flakes, graphene flakes, carbon nanotubes, and graphite powder. The current collector may have nickel flakes, carbon nanotubes, and graphite powder.

In some embodiments, the printed energy storage device can include an intermediate layer. The intermediate layer may be between the first electrode and the separator. The intermediate layer is between the second electrode and the separator.

At least one of the first electrode and the second electrode includes an ionic liquid. The separator can include the ionic liquid. The ionic liquid includes a cation selected from the group consisting of 1-ethyl-3-methylimidazolium, butyltrimethylammonium, 1-butyl-3-methylimidazolium, 1-methyl-3-propylimidazolium, 1-hexyl-3-methylimidazolium, choline, ethylammonium, tributylmethylphosphonium, tributyl(tetradecyl)phosphonium, trihexyl(tetradecyl)phosphonium, 1-ethyl-2,3-methylimidazolium, 1-butyl-1-methylpiperidinium, diethylmethylsulfonium, 1-methyl-3-propylimidazolium, 1-methyl-1-propylpiperidinium, 1-butyl-2-methylpyridinium, 1-butyl-4-methylpyridinium, 1-butyl-1-methylpyrrolidinium, and diethylmethylsulfonium, where the ionic liquid includes an anion selected from the group consisting of tetrafluoroborate, tris(pentafluoroethyl)trifluorophosphate, trifluoromethanesulfonate, hexafluorophosphate, tetrafluoroborate, ethyl sulfate, dimethyl phosphate, methansulfonate, triflate, tricyanomethanide, dibutylphosphate, bis(trifluoromethylsulfonyl)imide, bis-2,4,4-(trimethylpentyl) phosphinate, iodide, chloride, bromide, and nitrate.

In some embodiments, the ionic liquid can include 1-ethyl-3-methylimidazolium tetrafluoroborate (C2mimBF4).

In some embodiments, at least one of the first electrode, the separator, and the intermediate layer can include a salt. The salt includes an anion that is the same as the anion of the ionic liquid. In some embodiments, the salt can include zinc tetrafluoroborate and the ionic liquid can include 1-ethyl-3-methylimidazolium tetrafluoroborate.

In some embodiments, the salt can include zinc chloride. In some embodiments, the salt can include zinc bis(trifluoromethanesulfonyl)imide. In some embodiments, the salt can include zinc sulfate. In some embodiments, the salt can include zinc nitrate. In some embodiments, the salt can include zinc carbonate.

In some embodiments, the first electrode can include polyvinylidene difluoride.

In some embodiments, at least one of the first electrode, the second electrode, the separator, the intermediate layer, and the current collector can include a polymer selected from the group consisting of polyvinyl pyrrolidone (PVP), polyvinyl alcohol (PVA), polyvinylidene fluoride, polyvinylidene fluoride-trifluoroethylene, polytetrafluoroethylene, polydimethylsiloxane, polyethelene, polypropylene, polyethylene oxide, polypropylene oxide, polyethylene glycolhexafluoropropylene, polyethylene terefphtalatpolyacrylonitryle, polyvinyl butyral, polyvinylcaprolactam, polyvinyl chloride; polyimide polymers and copolymers (including aliphatic, aromatic and semi-aromatic polyimides), polyamides, polyacrylamide, acrylate and (meth)acrylate polymers and copolymers such as polymethylmethacrylate, polyacrylonitrile, acrylonitrile butadiene styrene, allylmethacrylate, polystyrene, polybutadiene, polybutylene terephthalate, polycarbonate, polychloroprene, polyethersulfone, nylon, styrene-acrylonitrile resin; polyethylene glycols, clays such as hectorite clays, garamite clays, organomodified clays; saccharides and polysaccharides such as guar gum, xanthan gum, starch, butyl rubber, agarose, pectin; celluloses and modified celluloses such as hydroxyl methylcellulose, methylcellulose, ethyl cellulose, propyl methylcellulose, methoxy cellulose, methoxy methylcellulose, methoxy propyl methylcellulose, hydroxy propyl methylcellulose, carboxy methylcellulose, hydroxy ethylcellulose, ethyl hydroxyl ethylcellulose, cellulose ether, cellulose ethyl ether, and chitosan.

In some embodiments, the second electrode can include the carbon nanotubes. In some embodiments, the second electrode can include a homogeneous paste including the carbon nanotubes and the ionic liquid. In some embodiments, the second electrode can include a conductive carbon. The conductive carbon can include graphite powder.

In some embodiments, the separator can have microspheres. The microspheres may include at least one of glass, alumina, silica, polystyrene, and melamine. The microspheres may be hollow. The microspheres may be solid. In some embodiments, the microspheres can have a diameter from about 0.5 microns to about 30 microns.

In some embodiments, the intermediate layer can include polyvinyl alcohol.

A conductive paste for a layer of a printed energy storage device, for example a printed zinc manganese-dioxide (Zn/MnO₂) battery, can include carbon nanotubes, and an ionic liquid, where the ionic liquid includes a cation selected from the group consisting of 1-ethyl-3-methylimidazolium, butyltrimethylammonium, 1-butyl-3-methylimidazolium, 1-methyl-3-propylimidazolium, 1-hexyl-3-methylimidazolium, choline, ethylammonium, tributylmethylphosphonium, tributyl(tetradecyl)phosphonium, trihexyl(tetradecyl)phosphonium, 1-ethyl-2,3-methylimidazolium, 1-butyl-1-methylpiperidinium, diethylmethylsulfonium, 1-methyl-3-propylimidazolium, 1-methyl-1-propylpiperidinium, 1-butyl-2-methylpyridinium, 1-butyl-4-methylpyridinium, 1-butyl-1-methylpyrrolidinium, and diethylmethylsulfonium, and where the ionic liquid includes an anion selected from the group consisting of tetrafluoroborate, tris(pentafluoroethyl)trifluorophosphate, trifluoromethanesulfonate, hexafluorophosphate, tetrafluoroborate, ethyl sulfate, dimethyl phosphate, methansulfonate, triflate, tricyanomethanide, dibutylphosphate, bis(trifluoromethylsulfonyl)imide, bis-2,4,4-(trimethylpentyl) phosphinate, iodide, chloride, bromide, and nitrate.

In some embodiments, the ionic liquid can include 1-ethyl-3-methylimidazolium tetrafluoroborate (C2mimBF4).

The carbon nanotubes may include single-wall carbon nanotubes. The carbon nanotubes may include multi-wall carbon nanotubes. The carbon nanotubes may be ground. In some embodiments, the carbon nanotubes and the ionic liquid can form a homogeneous mixture.

In some embodiments, the layer can be a first electrode. The first electrode can include manganese dioxide. In some embodiments, the first electrode can include graphite.

In some embodiments, a printed energy storage device can include a second electrode and a separator between the first electrode and the second electrode.

In some embodiments, the printed energy storage device can include an intermediate layer. The intermediate layer may be between the first electrode and the separator. The intermediate layer may be between the second electrode and the separator. In some embodiments, the printed energy storage device can include a current collector electrically coupled to the first electrode or the second electrode.

In some embodiments, at least one of the second electrode, the separator, and the intermediate layer can include the ionic liquid. In some embodiments, at least one of the second electrode, the separator, and the intermediate layer can include a salt. The salt includes a zinc salt. The salt includes an anion that is the same as the anion of the ionic liquid. The salt may include zinc tetrafluoroborate and the ionic liquid can include 1-ethyl-3-methylimidazolium tetrafluoroborate. In some embodiments, the salt can include zinc chloride. In some embodiments, the salt can include zinc bis(trifluoromethanesulfonyl)imide. In some embodiments, the salt can include zinc sulfate. In some embodiments, the salt can include zinc nitrate. In some embodiments, the salt can include zinc carbonate.

In some embodiments, at least one of the first electrode, the second electrode, the separator, the intermediate layer, and the current collector can have a polymer selected from the group consisting of polyvinyl pyrrolidone (PVP), polyvinyl alcohol (PVA), polyvinylidene fluoride, polyvinylidene fluoride-trifluoroethylene, polytetrafluoroethylene, polydimethylsiloxane, polyethelene, polypropylene, polyethylene oxide, polypropylene oxide, polyethylene glycolhexafluoropropylene, polyethylene terefphtalatpolyacrylonitryle, polyvinyl butyral, polyvinylcaprolactam, polyvinyl chloride; polyimide polymers and copolymers (including aliphatic, aromatic and semi-aromatic polyimides), polyamides, polyacrylamide, acrylate and (meth)acrylate polymers and copolymers such as polymethylmethacrylate, polyacrylonitrile, acrylonitrile butadiene styrene, allylmethacrylate, polystyrene, polybutadiene, polybutylene terephthalate, polycarbonate, polychloroprene, polyethersulfone, nylon, styrene-acrylonitrile resin; polyethylene glycols, clays such as hectorite clays, garamite clays, organomodified clays; saccharides and polysaccharides such as guar gum, xanthan gum, starch, butyl rubber, agarose, pectin; celluloses and modified celluloses such as hydroxyl methylcellulose, methylcellulose, ethyl cellulose, propyl methylcellulose, methoxy cellulose, methoxy methylcellulose, methoxy propyl methylcellulose, hydroxy propyl methylcellulose, carboxy methylcellulose, hydroxy ethylcellulose, ethyl hydroxyl ethylcellulose, cellulose ether, cellulose ethyl ether, and chitosan.

In some embodiments, at least one of the first electrode, the second electrode, the separator, and the current collector can include polyvinylidene difluoride.

In some embodiments, the current collector can include the carbon nanotubes.

In some embodiments, the current collector can have at least one of nickel flakes, graphene flakes, and graphite powder. The current collector may have nickel flakes, for example a current collector electrically coupled to the second electrode. The current collector may have graphene flakes, for example a current collector electrically coupled to the first electrode. The current collector may have nickel flakes and graphene flakes. The current collector may have nickel flakes, graphene flakes, and graphite powder. The current collector may have nickel flakes and carbon nanotubes. The current collector may have nickel flakes, graphene flakes, and carbon nanotubes. The current collector may have nickel flakes, graphene flakes, carbon nanotubes, and graphite powder. The current collector may have nickel flakes, carbon nanotubes, and graphite powder.

In some embodiments, the separator can have microspheres. The microspheres may include at least one of glass, alumina, silica, polystyrene, and melamine. The microspheres may be hollow. The microspheres may be solid. In some embodiments, the microspheres can have a diameter from about 0.5 microns to about 30 microns.

In some embodiments, the intermediate layer can include polyvinyl alcohol.

For purposes of summarizing the invention and the advantages achieved over the prior art, certain objects and advantages are described herein. Of course, it is to be understood that not necessarily all such objects or advantages need to be achieved in accordance with any particular embodiment. Thus, for example, those skilled in the art will recognize that the invention may be embodied or carried out in a manner that can achieve or optimize one advantage or a group of advantages without necessarily achieving other objects or advantages.

All of these embodiments, if not stated otherwise, are intended to be within the scope of the invention as defined by the claims. These and other embodiments will become readily apparent to those skilled in the art from the following detailed description having reference to the attached figures, the invention not being limited to any particular disclosed embodiment(s).

### BRIEF DESCRIPTION OF THE DRAWINGS

These and other features, aspects, and advantages of the present disclosure are described with reference to the drawings of certain embodiments, which are intended to illustrate certain embodiments and not to limit the invention.
Figure 1 is a cross-sectional or side elevational view of an example embodiment of a printed battery.
Figure 2 is a cross-sectional or side elevational view of another example printed battery.
Figure 3A is a photographic plan view of an example embodiment of a printed battery.
Figure 3B is a photographic side view of the printed battery of Figure 3A.
Figure 4 is a photographic perspective view of another example embodiment of a printed battery.
Figure 5 is a scanning electron microscope (SEM) image of a cross section of an example embodiment of a printed battery.
Figure 6 is a graph of battery potential versus time of discharge for the printed battery of Figures 3A and 3B.
Figure 7 is a cross-sectional or side elevational view of an example embodiment of a printed battery.
Figure 8 is a cross-sectional or side elevational view of another example embodiment of a printed battery.
Figure 9 is a SEM image of a cross section of an example embodiment of a printed separator.
Figure 10 is an example embodiment of a process for fabricating an example embodiment of a printed battery.
Figure 11 is a graph of battery potential versus time of discharge for an example printed battery.

### DETAILED DESCRIPTION

Although certain embodiments and examples are described below, those of skill in the art will appreciate that the invention extends beyond the specifically disclosed embodiments and/or uses and obvious modifications and equivalents thereof. Thus, it is intended that the scope of the invention herein disclosed should not be limited by any particular embodiments described below.

In some embodiments, a zinc-manganese dioxide(Zn/MnO₂) battery comprises all elements of which are printed. For example, all components of the battery, including current collectors, electrodes, battery separator, and leads may be sequentially printed onto one substrate. Printing can be a scalable, cost effective, and productive technique. For example, a battery having all components which are printed may facilitate a simplified manufacturing process, and/or facilitate fabrication of a battery having a desired form-factor with reduced costs. In some embodiments, a printed Zn/MnO₂ battery separator can include a solid state electrolyte. In some embodiments, a printed a Zn/MnO₂ battery includes a printed electrolyte, for example an electrolyte printed onto a previously printed battery separator for providing a printed battery having a liquid electrolyte. For example, all components of the battery, including the electrolyte, can be sequentially printed onto a substrate.

In some embodiments, printed Zn/MnO₂ batteries can have a thickness from about 0.1mm to about 0.4 mm and can be flexible depending on the substrate that at least some of the battery layers are printed on. Printed zinc-manganese dioxide batteries can be used as a separate device or integrated on a substrate with other electronic components, such as light-emitting diode (LED) lights. Devices into which printed zinc-manganese dioxide batteries may be integrated devices can be thin and/or flexible. An integrated printed zinc-manganese dioxide battery may not need additional connection elements like wires for electrical connection with other electronics as all necessary connections may also be printed.

A fully printed battery can enable fabrication of batteries having a variety of shapes. In some embodiments, a printed battery can be printed around other components of an integrated device and/or printed on a substrate with an unusual shape. For example, the printed battery may be printed on commercially available substrates (e.g., polyimide film such as Kapton® from DuPont, Inc. of Wilmington, Delaware) or manufactured. In some embodiments, one printed battery can be printed above one or more other energy storage devices, including, for example, one or more other printed batteries. For example, the printed battery can be connected with one or more other printed batteries in parallel to enable an increased energy storage capacity per unit of area and/or in series to enable an increased working voltage. Suitable zinc (Zn) for a printed battery may be commercially available (e.g., from Teck American Inc., of Spokane, Washington) or manufactured. Suitable manganese dioxide (MnO₂) may be commercially available (e.g., from Minera Autlan, of Mexico) or manufactured.

In some embodiments, a printed Zn/MnO₂ battery has an open circuit potential from about 1.5 volts(V) to about 1.55V and a capacitance of about 0.1 mAh/cm² when discharged at about 0.1 mA/cm². For example, three 1×1.5 inch printed zinc-manganese dioxide batteries connected in series printed on a substrate with 30 blue micro-light-emitting-diodes (microLEDs) can light the microLEDs non-stop for 1.5 hours. In some embodiments, printed batteries can be integrated into thin and/or small consumer electronic devices for powering the devices. For example, printed batteries can power lights and/or audio modules of greeting and/or business cards, thin advertisement tools like magazine inserts, skin care products, safe pass cards, and medical devices. An on-off switch (e.g., a press-button control) for the LEDs can further extend the operating life of batteries.

In some embodiments, a printed zinc-manganese dioxide includes an electrolyte comprising ionic liquid 1-ethyl-3-methylimidazolium tetrafluoroborate (C₂mimBF₄). The ionic liquid may be commercially available (e.g., IoLiTec Ionic Liquids Technologies GmbH, of Heilbronn, Germany) or manufactured. In certain embodiments, the electrolyte for Zn/MnO₂ batteries may comprise a "green" electrolyte - ionic liquid 1-ethyl-3-methylimidazolium tetrafluoroborate (C₂mimBF₄). Some existing Zn/MnO₂ batteries (comprising zinc carbon and zinc alkaline) have either an aqueous solution of ammonium and zinc chlorides or an aqueous solution of potassium hydroxide as electrolytes. Aqueous electrolytes can evaporate easily, including for example after integration into a battery, and special measures may need to be taken to inhibit or prevent evaporation or leakage. A battery having an aqueous electrolyte may require special care during battery assembly. C₂mimBF₄ is a non- volatile electrolyte. Non-volatile electrolytes may be suitable for printing processes. For example, an electrolyte may maintain or substantially maintain a concentration during a battery production process and/or in post assembly life of a battery comprising the electrolyte. Ionic liquids are ecological "green" electrolytes in terms that they do not contaminate air. Another attractive property of the ionic liquid used is non-flammability. For example, an ionic liquid electrolyte cannot self-ignite during a battery overload or shortage, and will not support any flame.

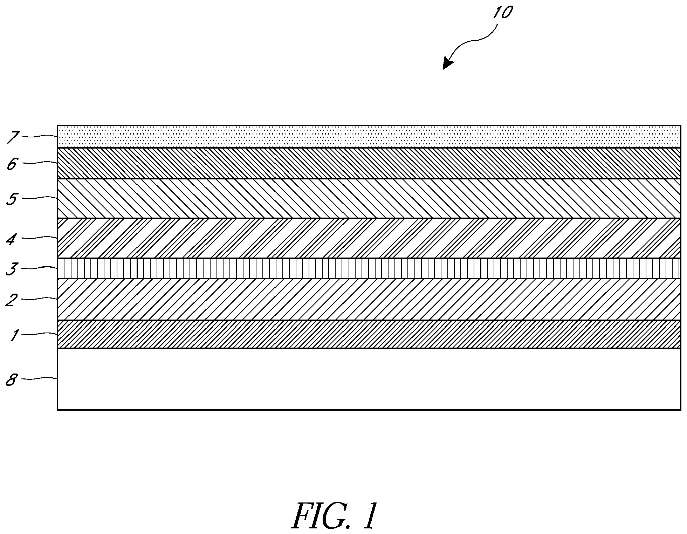

Figure 1 is a cross-sectional or side elevational view of an example embodiment of the a printed battery 10, for example a zinc-manganese dioxide battery. The printed battery 10 includes a first current collector 1, a first electrode layer 2, an intermediate layer 3, a separator layer 4, a second electrode layer 5, a second current collector 6, an insulator layer 7, and a substrate 8. The first current collector 1 is configured provide electrical contact between the first electrode layer 2 and an external circuit. In some embodiments, the printed battery 10 may optionally not include the intermediate layer 3. In some embodiments, the current collector 6 is configured to provide electrical contact between the second electrode layer 5 and an external circuit. In some embodiments, the printed battery 10 may optionally not include the current collector 1, for example in embodiments in which the substrate 8 comprises a material having conductivity allowing connection to an external circuit. In some embodiments, the intermediate layer 3 comprises a plurality of layers. In some embodiments, the printed battery 10 may optionally not include the intermediate layer 3.

In some embodiments, the printed battery 10 can be printed layer by layer. For example, layers 1-7 of the printed zinc manganese dioxide battery 10 may be printed one above the other in the following sequence: the first current collector layer 1 may be printed onto a surface of the substrate 8; the first electrode layer 2 may be printed onto a surface of the first current collector layer 1; the intermediate layer 3 may be printed onto a surface of the first electrode layer 2; the separator layer 4 may be printed onto a surface of the intermediate layer 3, or as described herein onto a surface of the first electrode layer 2;, the second electrode layer 5 may be printed onto a surface of the separator layer 4; the second current collector layer 6 may be printed onto a surface of the second electrode layer 5; and the insulator layer 7 may be printed onto a surface of the current collector layer 6. The insulator layer 7 may comprise a polymer and may provide the printed battery 10with a seal (e.g., a hermetic seal).

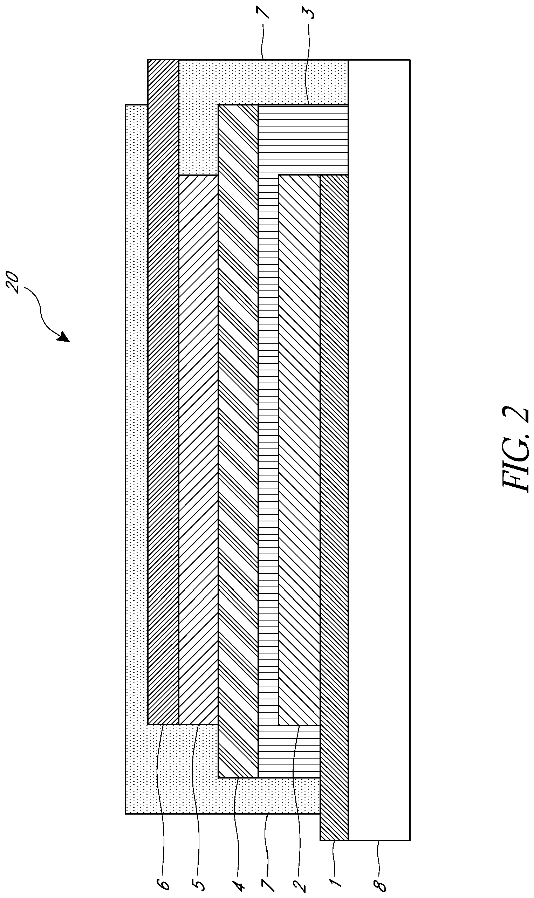

Figure 2 is a cross-sectional or side elevational view of another example embodiment of a printed battery 20, for example a zinc-manganese dioxide battery comprising the layers 1-8 discussed herein. The current collector layers 1, 6 may extend beyond a portion of electrode layers 2, 5, for example to facilitate coupling with an external circuit. The intermediate layer 3 may form an enclosure over at least a portion of electrode layer 2, for example to facilitate separation between the two electrode layers 2, 5. The polymer insulator layer 7 may form an enclosure around the electrode layers 2, 5, the intermediate layer 3, and the separator layer 4, for example to seal the layers of the printed battery 10 (e.g., with a hermetic seal).

In some embodiments, a zinc anode (e.g., the first electrode layer 2 in Figure 1) may comprise a polymer binder (e.g., high molecular weight polyvinylidene difluoride polymer), zinc, and/or an electrolyte comprising zinc salt such as zinc tetrafluoroborate (ZnBF₄) and C₂mimBF₄ ionic liquid. The zinc salt has a common anion with the used ionic liquid and can create an electroactive couple with zinc (Zn/Zn²⁺).

In some embodiments, the separator layer 4 can comprise an electrolyte/polymer gel and microspheres (e.g., micro glass spheres as described in U.S. Patent App. No. 13/223,279, entitled "Printable Ionic Gel Separation Layer for Energy Storage Devices,"). For example, a printed zinc manganese-dioxide battery may comprise a separator layer including an electrolyte comprising ZnBF₄/C₂mimBF₄, a polymeric gel comprising copolymers of polyvinylidene difluoride, and micro glass spheres. Micro glass spheres may be commercially available (e.g., from Potters Industries, of Brownwood, Texas) or manufactured. Some commercially available batteries, including flexible batteries, use a separate sheet of membrane as a separator between electrode layers. A separator comprising solid microspheres is that the separator layer 4 may advantageously be printed. For example, the separator layer 4, including solid microspheres may be fabricated through a printing process, along with other components of a printed battery 10, instead of being formed during a separate fabrication process and then being integrated into the battery. Ionic conduction through the separator layer 4 may be realized by the ionic liquid/polymer gel. The polymer gel can be fabricated from polyvinylidene difluoride copolymer. The solid microspheres can enable the separator layer 4 to withstand applied pressure during the printing process, including for example subsequent printing of one or more other layers 5-7 of the printed battery 10 and, therefore, inhibit or prevent shorting of the printed battery 10. A printed battery 10 including a printed separator layer 4 comprising solid microspheres can provide larger or significantly larger charge storage areas than batteries including a non-printed separator, for example because the separator layer 4 can be printed over a large surface area and/or have unique lateral shapes.

A printed battery 10 may optionally comprise the intermediate layer 3. The intermediate layer 3 may be an ultrathin layer (e.g., having a thickness in a range from about 1 micron (µm) to about 3 microns) that coats an underlying layer, for example the first electrode layer 2. The printed battery 10 may include an intermediate layer between other layers described herein, including, for example, between the separator layer 4 and the second electrode layer 5. The intermediate layer 3 can provide a smoother interface between two adjacent printed layers and/or help preserve the structural integrity of one or more underlying layers from damage due to pressure applied during printing of one or more subsequent layers (e.g., during printing of the separator layer 4). The intermediate layer 3 may also promote adhesion between the two adjacent layers, such as between the separator layer 4 and the first electrode layer 2. In some embodiments, the intermediate layer 3 comprises a polymeric gel and an electrolyte for ionic conduction. The electrolyte may comprise an ionic liquid, such as the same ionic liquid as the separator layer 4. The polymeric gel can be made from polyvinyl alcohol (PVA) polymers having different molecular masses. For example, the intermediate layer 3 may include an electrolyte comprising ZnBF₄/C₂mimBF₄ and a polymeric gel comprising polyvinyl alcohol. Other polymers and/or electrolytes may also be suitable.

In some embodiments, an electrode layer (e.g., the first electrode layer 2, the second electrode layer 5) can include carbon nanotubes (CNT). For example, a printed zinc manganese-dioxide battery cathode (e.g., the second electrode layer 5 of the printed battery 10) may comprise MnO₂, conductive carbon (e.g., graphite), a polymer (e.g., high molecular weight polyvinylidene difluoride polymer) as a binder, an electrolyte (e.g., an ionic liquid), and carbon nanotubes. Suitable graphite may be commercially available (e.g., from TIMCAL Ltd., of Westlake, Ohio) or manufactured. The printed zinc manganese-dioxide battery cathode may comprise a dispersion comprising ground carbon nanotubes and an ionic liquid (e.g., C₂mimBF₄). Carbon nanotubes may include single-wall (SWCNT) and/or multi-wall carbon nanotubes (MWCNT). Carbon nanotubes may be commercially available (e.g., from SouthWest NanoTechnologies Inc., of Norman, Oklahoma) or manufactured. The second electrode layer 5 may include a homogeneous paste comprising an ionic liquid and carbon nanotubes. Incorporation of carbon nanotubes into an electrode may improve electron conductivity within the electrode and/or facilitate incorporation of ionic liquid electrolyte in the electrode.

The composition of the current collector layers 1 and 6 may differ depending on the functions that each is designed to fulfill. The first current collector layer 1, for example configured to be at the bottom of a printed stack and/or to be electrically coupled to an anode, may comprise a mixture of nickel (Ni) flakes and a polymer, and may be printed on the substrate 8 to provide good adherence of the first electrode layer 1 to the substrate 8. Nickel flakes may be commercially available (e.g., from Novamet Specialty Products Corp. of Wyckoff, New Jersey) or manufactured. The second current collector layer 6, for example configured to electrically couple to a cathode, may comprise graphene flakes and a polymer, and may be printed over the second electrode layer 5. Graphene flakes may be commercially available (e.g., from XG Sciences, Inc. of Lansing, Michigan) or manufactured. Graphene particles in the second current collector layer 6 are generally light and bendable such that they do not penetrate through the second electrode layer 5 during printing of the second current collector layer 6.

Example combinations of conductive materials for current collectors comprise:
1) Ni flakes
2) Graphene flakes
3) Ni and graphene flakes
4) Ni flakes, graphene and graphite powder
5) Ni flakes, CNTs
6) Ni flakes, graphene, CNTs
7) Ni flakes, graphene, CNTs, graphite powder
8) Ni flakes, CNTs, graphite powder
9) Graphene, CNTs
10) Graphene, CNTs, graphite powder.

A polymer insulator layer 7, such as a hermetic printed layer, may optionally be used to seal the printed battery 10, for example to inhibit or prevent contact between the atmosphere (e.g., water and oxygen) with the materials of the printed battery 10. The insulator layer 7 may comprise, for example, an environmentally robust polymer.

### Battery Performance Measurement

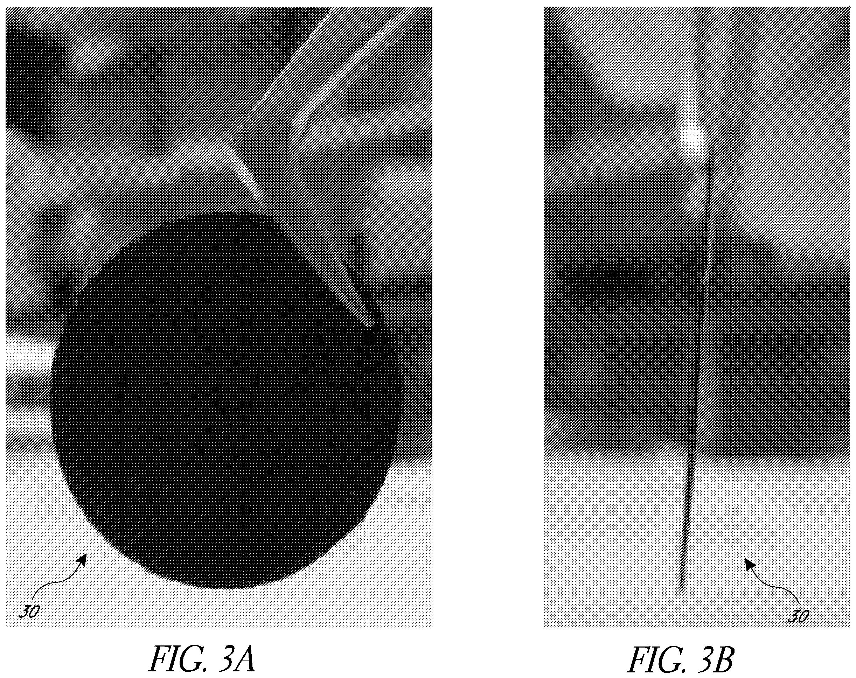

Printed zinc manganese dioxide (Zn/MnO₂) batteries may be printed in different designs depending on testing procedure. The batteries discharged in electrochemical cells can be printed on aluminum (Al) foil without current collectors. Figures 3A and 3B illustrate an example printed Zn/MnO₂ battery on Al foil. Figure 3A is a photographic plan view of an example embodiment of a printed battery, and Figure 3B is a photographic side view of the printed battery of Figure 3A. The electrochemical cell can then connect to a potentiostat.

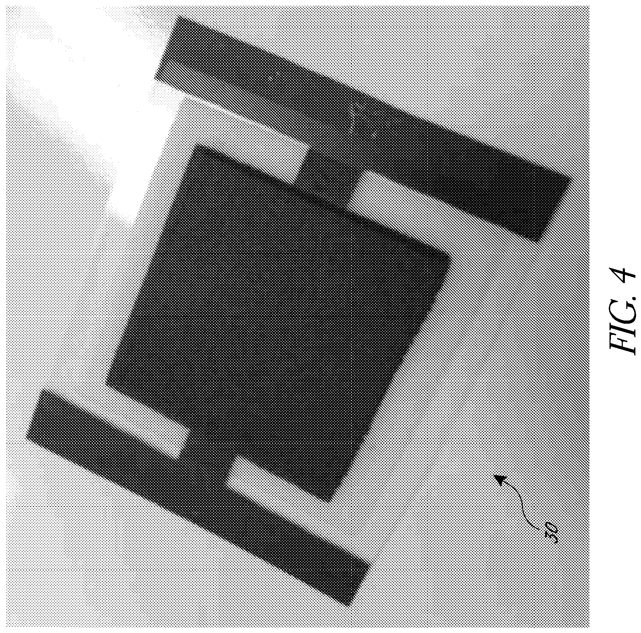

Figure 4 is a photographic perspective view of another example of a printed battery 40. The printed battery 40 shown in Figure 4 is a Zn/MnO₂ battery including a polyethylene terephthalate (e.g., Mylar®) substrate and including bus bars for connecting to a potentiostat.

### Example Compositions of Printed Battery Layers; not according to the invention

An example first electrode layer 2 comprises, by weight:
high molecular weight polyvinylidene difluoride polymer (PVDF, e.g., KYNAR® HSV 900 from Arkema, Inc. of King of Prussia, Pennsylvania) - 0.6%
Zn powder (particle size below 10 microns) - 99.31%
1 mol/L (M) ZnBF₄ in C₂mimBF₄ electrolyte - 0.09%

An example intermediate layer 3 comprises, by weight:
PVA 133,000 molecular weight - 6.86%
PVA 6,000 molecular weight - 35.73%
1 M ZnBF₄ in C₂mimBF₄ electrolyte - 57.41%

An example separator layer 4 comprises, by weight:
PVDF (e.g., KYNAR® ADX 161 from Arkema, Inc., of King of Prussia, Pennsylvania) - 3.56%
1 M ZnBF₄ in C₂mimBF₄ electrolyte - 36.96%
Glass spheres (less than 20 microns in diameter) - 59.48%

An example second electrode layer 5 comprises, by weight:
high molecular weight PVDF HSV 900 - 4.89%
MWCNT - 0.8%
C₂mimBF₄ ionic liquid - 4.51 %
Graphite powder - 2.35%
MnO₂ - 87.05%

An example first current collector layer 1 comprises, by weight:
high molecular weight PVDF HSV 900- 5.41%
Ni flakes - 94.49%

An example second current collector layer 6 comprises, by weight:
high molecular weight PVDF HSV 900 - 17.42%
Graphene flakes - 82.58%

An example insulator layer 7 comprises, by weight:
high molecular weight PVDF HSV 900 - 100%

### Example Printable Ink Compositions (examples of successful ink compositions), Preparation Process, Properties, and Curing Conditions; the examples are not according to the invention

An example composition of an ink for a first electrode layer 2 comprises, by weight:
high molecular weight PVDF HSV 900- 0.51%
Zn powder (particle size below 10 microns) - 85.12%
1-methyl-2-pyrrolidinone (MP) solvent - 14.29%
1 M ZnBF₄ in C₂mimBF₄ electrolyte - 0.08%

An example procedure to prepare the ink for the first electrode layer 2 includes:
Prepare PVDF HSV 900 base: Heat MP to 60 °C and progressively add PVDF HSV 900. Mix for 30 minutes at 60 °C using a laboratory egg.
Prepare electrolyte: Dissolve ZnBF₄ in C₂mimBF₄ by mixing at 50 °C in a laboratory egg until the zinc salt is dissolved.
Take PVDF HSV 900 base, electrolyte, and MP and sonicate for 10 minutes.
Take the sonicated mixture, preheat to 40 °C, and add additional MP and zinc dust. Stir for 30 minutes using a laboratory egg.

The ink for the first electrode layer 2 fabricated using the example method can have a viscosity of about 10,000 centipoise (cP). An example curing profile for this composition is at a temperature of 130 °C for between 3 and 5 minutes.

An example composition for an ink for an intermediate layer 3 comprises, by weight:
MP solvent - 81.19%
PVA 133,000 molecular weight - 1.29%
PVA 6,000 molecular weight - 6.72%
1 M ZnBF₄ in C₂mimBF₄ electrolyte - 10.8%

An example procedure to prepare the ink for the intermediate layer 3 includes:
Preheat MP to 80 °C. Slowly pour PVA 133,000 molecular weight into MP. Mix using magnetic bar.
Add PVA 6,000 molecular weight to the MP solution when the PVA 133,000 is dissolved.
Reduce heat to 60 °C and add ZnBF₄ in C₂mimBF₄ electrolyte drop wise. Stir using laboratory egg. Cool down the mixture until gelled.

The ink for the intermediate layer 3 fabricated using the example method can have a viscosity of about 100 cP. An example curing profile for this composition is at a temperature of 130 °C for between 5 and 7 minutes.

An example composition of an ink for the separator layer 4 comprises, by weight:
PVDF ADX 161 - 2.92%
1 M ZnBF₄ in C₂mimBF₄ electrolyte - 30.34%
Glass spheres (less than 20 microns in diameter) - 48.82%
MP solvent - 17.92%

An example procedure to prepare the ink for the printed separator layer 4 includes:
Prepare PVDF ADX 161 base: Preheat MP to 80 °C. Add PVDF ADX 161. Mix until PVDF ADX 161 is dissolved using a laboratory egg.
Preheat PVDF ADX 161 base to 60 °C and add ZnBF₄ in C₂mimBF₄ electrolyte drop by drop while mixing using a laboratory egg.
Cool down the mixture and add glass spheres. Mix for 10 minutes at room temperature using a laboratory egg.

The ink for the separator layer 4 fabricated using the example method can have a viscosity of about 13,OOOcP. An example curing profile for this composition is at a temperature of 130 °C for between 5 and 7 minutes.

An example composition for an ink for a second electrode layer 5 comprises, by weight:
high molecular weight PVDF HSV 900 - 2.3%
MWCNT - 0.38%
C₂mimBF₄ ionic liquid - 2.13%
MP solvent - 52.79%
Graphite powder - 1.11%
MnO₂ - 41.1%

An example procedure to prepare the ink for the second electrode layer 5 includes:
Prepare MWCNTs paste in C₂mimBF₄: Grind a mixture of 15% of the MWCNTs and 85% of the C₂mimBF₄ in a mortar and pestle in glove box for 5 minutes, then grind in automated mortar and pestle for 1 hour.
Prepare PVDF HSV 900 base: Heat MP to 60 °C and progressively add PVDF HSV 900. Mix for 30 minutes at 60 °C using a laboratory egg.
Take PVDF HSV 900 base and mix with MWCNT paste under sonication and at 50 °C for 30 minutes. Add graphite powder and MnO₂ and mix at 70 °C for 90 minutes using a laboratory egg.

The ink for the second electrode layer 5 fabricated using the example method can have a viscosity of about 9,000 cP. An example curing profile for this composition is at a temperature of 130 °C for between 3 and 5 minutes.

An example composition for an ink for the first current collector layer 1 comprises, by weight:
PVDF HSV 900 - 3.63%
Ni flakes - 63.47%
MP solvent - 32.9%

An example procedure to prepare the ink for the first current collector layer 1 includes:
Prepare PVDF HSV 900 base: Heat MP to 60 °C and progressively add PVDF HSV 900. Mix for 30 minutes at 60 °C using a laboratory egg. Add Ni flakes to the PVDF HSV 900 base while continuing to stir.

An example curing profile for the ink for the first current collector layer 1 fabricated using the example is at a temperature of 130 °C for between 3 and 5 minutes.

An example composition for an ink for the second current collector layer 6 comprises, by weight:
PVDF HSV 900 - 3.24%
Graphene flakes - 15.68%
MP solvent - 81.08%

An example procedure to prepare the ink for the second current collector layer 6 includes:
Prepare PVDF HSV 900 base: Heat MP to 60 °C and progressively add PVDF HSV 900. Mix for 30 minutes at 60 °C using a laboratory egg. Disperse graphene flakes in MP using ultrasonic bath (15 minutes). Add the grapheme-MP dispersion to the PVDF HSV 900 base while keeping stirring for another 60 minutes.

An example curing profile for the ink for the second current collector layer 2 fabricated using the above example is at a temperature of 130 °C for between 3 and 5 minutes.

An example composition of a polymer insulator layer comprises, by weight:
PVDF HSV 900 - 10%
MP solvent - 90%

A higher percentage of PVDF HSV 900 would result in higher viscosity and a lower percentage of PVDF HSV 900 would result in a lower viscosity, which can affect the thickness of a printed layer. Printed layers are generally desired to be as thin as possible, but still able to perform their intended function, such as acting as an environmental barrier for an insulator layer 7.

An example procedure to prepare the ink for the insulator layer 7 includes:

Heat MP to 60 °C and progressively add PVDF HSV 900. Mix for 30 minutes at 60 °C using a laboratory egg. An example curing profile for the ink for the insulator layer 7 fabricated using the above example is at a temperature of 130 °C for between 3 and 5 minutes

### Example Printed Battery Physical Parameters

### Example Thicknesses of the Printed Layers

The current collector layer 1 may have a thickness in a range from about 2 µm to about 5 µm.

A zinc (Zn) electrode layer (e.g., the first electrode layer 2) may have a thickness in a range from about 20 µm to about 70 µm, for example depending on a material of the substrate 8 and absence or presence of the current collector layer 1.

The intermediate layer 3 may have a thickness in a range from about 1 µm to about 3 µm.

The separator layer 4 may have a thickness in a range from about 10 µm to about 30 µm.

A MnO₂ electrode layer (e.g., second electrode layer 5) may have a thickness in a range from about 20 µm to about 60 µm.

The second current collector layer 6 may have a thickness in a range from about 5 µm to about 7 µm.

The insulator layer 7 may have a thickness of about 10 µm.

The total thickness of a fully printed battery including the layers 1-7 may have a thickness in a range from about 70 µm to about 200 µm.

The substrate 8 can have thickness in a range from about 10 µm to about 200µm, making a maximum thickness of the device about 400 µm. On thin substrates 8 (e.g., substrates 8 having a thickness of about 30 µm to about 60 µm), the total thickness of a fully printed battery including the layers 1-7 can be as thin as about 130 microns.

### Physical Characteristics of Printed Zn/MnO₂ Battery

An example of printed battery 30 on an Al substrate is shown in Figures 3A and 3B. The example printed battery 30 has a round shape (e.g., having a diameter of about 18 mm) and has been cut out from the Al substrate. The overall thickness of the battery 30 (including the Al substrate) is about 200 microns. The example battery 30 has a total weight of about 0.137 grams (g). Approximately 2/3 of the total weight is the weight of the 60 microns thick Al substrate.

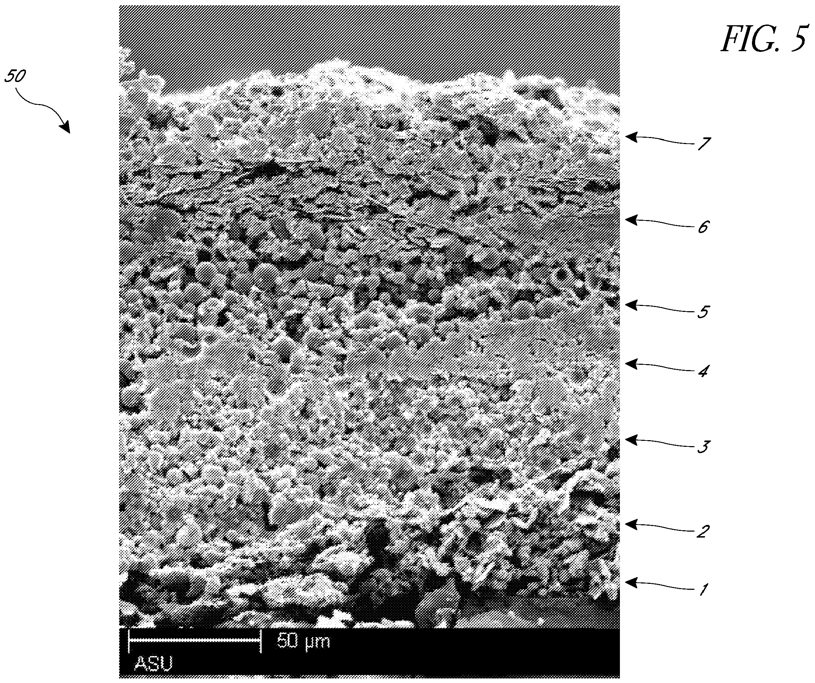

Figure 5 shows SEM image of a cross-section of an example printed battery 50. The printed battery 50 shown in Figure 5 is a Zn/MnO₂ battery. The example printed battery was cracked under liquid nitrogen and then imaged. A carbon foam (not shown) was used as a substrate for convenience of the "crack" preparation. The printed Zn/MnO₂ battery 50 printed on a carbon foam substrate includes: a carbon foam substrate structure; a thin layer of Ni current collector 1; a Zn anode layer 2 (Zn spheroid particles are visible in Figure 5); a very thin intermediate layer 3; a glass sphere separator layer 4; a MnO₂ layer 5; and a thin graphene current collector layer 6.

### Details of the battery performance

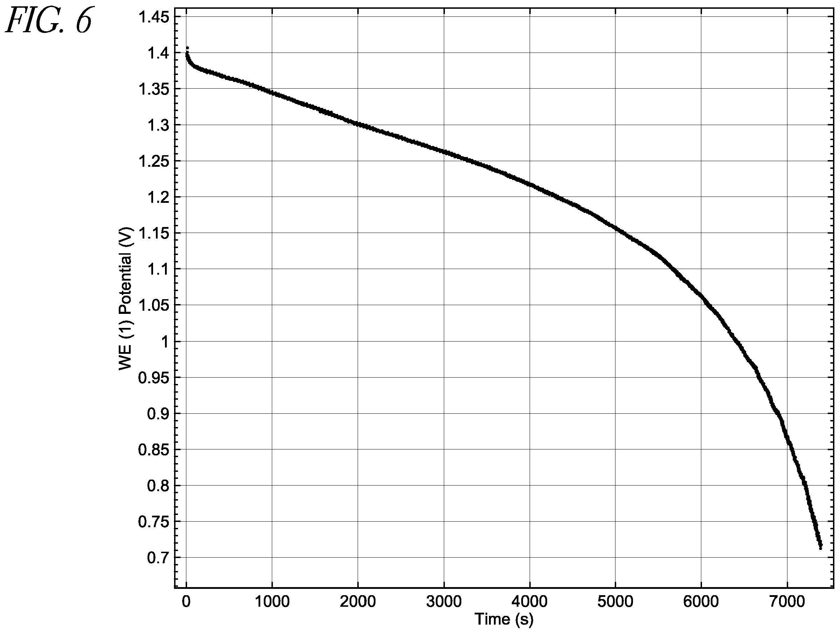

Figure 6 is a graph of the dependence of battery potential versus time of discharge for the printed battery 30 of Figures 3A and 3B at constant current of about 0.06 mA/cm², or a constant current discharge curve The printed battery 30 was discharged with the current of 0.158 milliAmperes (mA) and lasted 1.9 hours. The cut-off voltage for the calculations was 0.9 V. The capacity of the printed Zn/MnO₂ battery 30 is 0.12 mAh/cm² at moderate current drain of 0.06mA/cm². The open circuit potential of the printed battery 30 is 1.5 V and the working voltage is about 1.25 V.

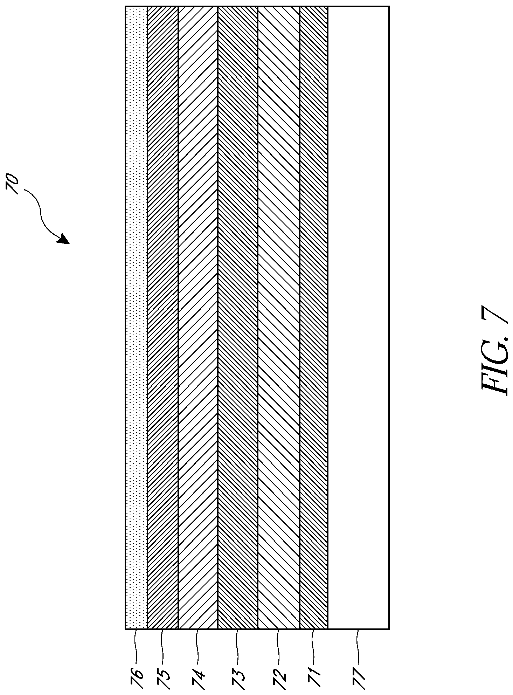

Figure 7 shows a cross sectional or side elevational view of an example embodiment of a printed battery 70. The printed battery 70 can include a substrate 77, an optional first current collector layer 71 over the substrate 77, a first electrode layer 72 over the first current collector layer 71, a separator layer 73 (e.g., a separator including an electrolyte) over the first electrode layer 72, a second electrode layer 74 over the separator layer 73, and a second current collector layer 75 over the second electrode layer 74. In some embodiments, the printed battery 70 can include a separator sealing layer (not shown) beneath and/or above the separator layer 73. The separator sealing layer may facilitate sealing of the separator layer 73, and/or adhesion of the separator layer 73 to one or more adjacent printed layers (e.g., the first electrode layer 72 and/or the second electrode layer 74). In some embodiments, the separator sealing layer can have a same or similar composition and/or thickness as the intermediate layers described herein (e.g., intermediate layer 3 as shown in Figure 1, having a thickness of less than about 1 micrometer (µm)). In some embodiments, each layer can be printed sequentially (e.g., one layer over the previous layer) for forming the battery 70. In some embodiments, an optional insulator layer 76 can be printed over the second current collector layer 75 to facilitate sealing of the battery 70 (e.g., for providing a hermetic seal for the printed battery 70). In some embodiments, the first electrode layer 72 can be printed onto the substrate 77, for example without the first current collector layer 71, if the substrate 77 comprises or is an electrically conductive material. In some embodiments, the first current collector 71 and/or the second current collector 75 can have a similar or same composition as other current collectors described herein (e.g., the current collectors 1, 6 of Figure 1). In some embodiments, example embodiments of the first current collector 71 and/or the second current collector 75 can include nickel, for example being made from a conductive nickel-containing film. Examples of suitable nickel-containing films are provided in pages 21-23 and 25-33 of U.S. Provisional Patent Application No. 61/747,093, filed December 28, 2012.

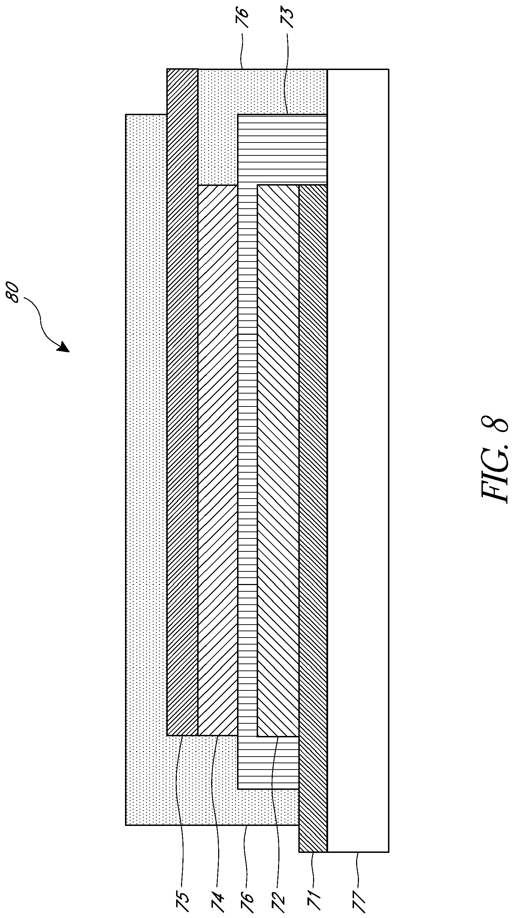

Figure 8 is a cross-sectional or side elevational view of another embodiment of a printed battery 80, for example a battery 80 comprising the layers 71-76 shown in Figure 7. In some embodiments, the printed battery 80 can include a separator sealing layer (not shown) beneath and/or above the separator layer 73. The battery 80 may be formed over a substrate 77. The current collector layers 71, 75 may laterally extend beyond the electrode layers 72, 74, for example to facilitate coupling with an external circuit. The separator layer 73 may form an enclosure over at least a portion of first electrode layer 72, for example to facilitate separation between the two electrode layers 72, 74. The insulator layer 76 may form an enclosure around the electrode layers 72, 74 and the separator layer 73, for example to seal the layers of the printed battery 80 (e.g., with a hermetic seal). In some embodiments, the battery 80 is a zinc-manganese dioxide (Zn/MnO₂) battery, the first electrode layer 72 comprising a zinc electrode and the second electrode layer 75 comprising a manganese dioxide (MnO₂) electrode. In some embodiments, the separator layer 73 can include a component that provides structural integrity to the separator layer 73 (e.g., a plurality of glass spheres, such as glass microspheres as described herein). In some embodiments, the separator layer 73 can be impregnated (e.g., soaked) with an electrolyte. In some embodiments, the insulator layer 76 can comprise or be made of a polymer insulator material for sealing the battery 80 (e.g., for providing a hermetic seal).

In some embodiments, a printed separator layer (e.g., the separator layer 73 of Figure 7, the separator layer 73 of Figure 8) is porous. As described herein, a separator layer may include components that provide structural support to the separator layer, such as microspheres (e.g., solid supports such as glass particles having a spherical or substantially spherical shape). A separator layer including structural support components may advantageously demonstrate improved ability to maintain a desired separation between electrodes of the battery (e.g., to inhibit, prevent, or substantially prevent an electrical short in the battery), for example during the application of pressure to components of the battery such as during printing subsequent layers of the battery. The separator layer can be printed on and/or over a previously printed battery layer, for example facilitating fabrication of a printed battery having all layers which are printed (e.g., sequentially printed on a substrate). Additional layers of the printed battery can be printed on and/or above the printed separator layer subsequently. In some embodiments, the porosity of the separator layer may advantageously facilitate holding of a liquid electrolyte by the separator (e.g., a liquid electrolyte which can be printed in a process subsequent to the process for printing of the separator layer). For example, a separator layer including pores that can provide a direct or substantially direct path from a first surface of the separator (e.g., a surface adjacent a first electrode) to an opposite second surface of the separator (e.g., a surface adjacent to a second electrode), and holding a liquid electrolyte, may facilitate improved ionic conductance between electrodes of the battery. In some embodiments, a separator layer including pores having a size between about 0.1 microns and about 10 microns (e.g., about 1 micron). Pore size may depend, for example, on at least one of polymer concentration, filler (e.g., microsphere) concentration, filler type, and/or filler size. The pores may be filled with a liquid electrolyte can provide improved ionic conductance between a first electrode layer and a second electrode layer, for example having an improved ionic conductivity as compared to a separator having a solid state electrolyte (e.g., an electrolyte which is included in a mixture for preparing the separator layer, and where the mixture is cured during the battery fabrication process for removing one or more solvents of the mixture, such as during the fabrication process of the separator layer).

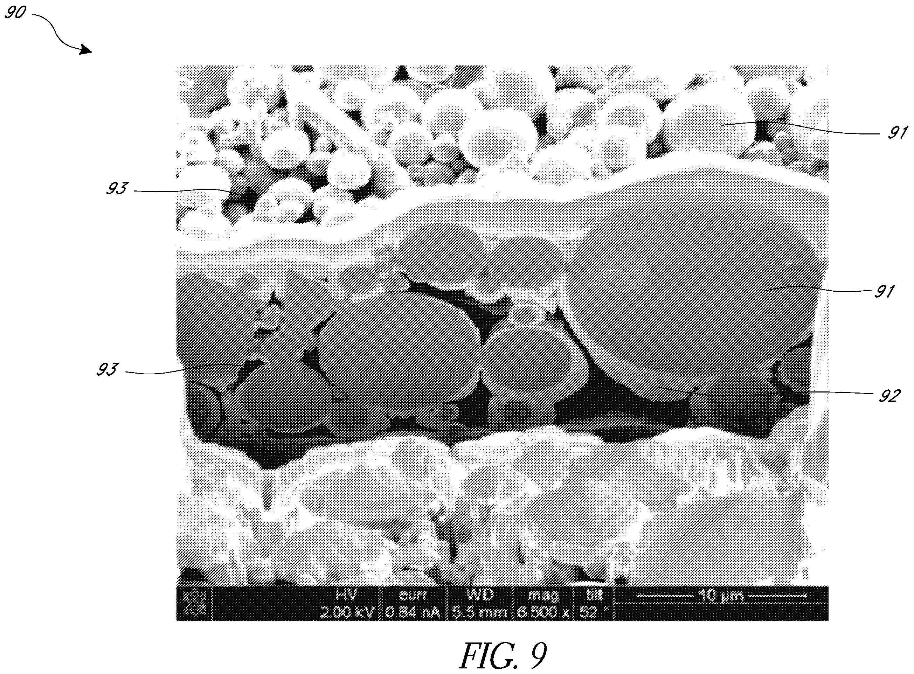

Figure 9 shows a tilt SEM image of an example embodiment of a printed separator layer 90, the SEM image showing both an angled top view and a cross-section view of the printed separator layer 90. In the top view, spherical structures 91 (e.g., glass microspheres that may be at least partially coated with polymer) are shown, although coated with platinum to enhance visibility. The spherical structures 91 can provide structural support for the separator layer 90. Pores 93 (e.g., pores resulting from one or more solvents evaporating during curing of the separator layer ink) are visible between the spherical structures 91. The pores 93 may result from evaporation of solvent in the ink used to print the separator layer 90, for example after printing and/or after curing. The pores 93 have irregular sizes, including some dimensions having a size between about 0.1 microns and about 10 microns. As described herein, an electrolyte can be held in one or more pores 93 of the printed separator layer 90, for example after seeping into the pores upon printing of an electrolyte layer on and/or over the separator layer 90, and/or upon heating of an electrolyte layer previously printed on and/or over the separator layer 90. Spherical structures 91, polymer 92, and a plurality of pores 93 can be seen in the cross-sectional view. The polymer 92 at least partially coats at least some of the spherical structures 91. Polymer coverage can at least partially depend, for example, on polymer type, filler type, polymer concentration, filler concentration, and/or solvent type.

In some embodiments, a printed battery (e.g., the printed battery 70 and/or the printed batter 80) includes a printed electrolyte. The electrolyte may be printed on a printed separator layer (e.g., separator layer 73 of Figure 7, separator layer 73 of Figure 8, separator layer 90 of Figure 9). In some embodiments, the printed electrolyte has no or substantially no other additives, for example no additives in addition to an ionic liquid and a salt. For example, the electrolyte can have sufficient viscosity for printing directly onto a separator layer. In some embodiments, the printed electrolyte diffuses into the pores of the separator layer. For example, the electrolyte can fill or substantially fill the pores of the porous separator layer. In some embodiments, all or substantially all of the printed electrolyte diffuses into the pores of the printed separator layer prior to the printing of a subsequent layer of the printed battery. In some embodiments, after printing, the printed electrolyte layer is heated to facilitate reduction in the viscosity of the printed electrolyte layer, facilitating diffusion of the electrolyte into the pores of the separator layer. For example, heating of the printed electrolyte layer can hasten the process of filling the pores of the separator layer with the electrolyte. For example, no or substantially no electrolyte remains above the separator layer upon completion of the process in which at least the electrolyte layer is heated, when a second electrode layer (e.g., second electrode layer 74 of Figure 7, second electrode layer 74 of Figure 8), and/or a separator sealing layer is printed on the separator layer. Little or none of the electrolyte layer may evaporate during the heating and diffusion process.

In some embodiments, an amount of electrolyte printed on a separator layer can depend at least partially on, for example, separator layer thickness and/or separator layer porosity (e.g., pore size, number of pores). As described above, the separator layer porosity can at least partially depend on separator layer polymer concentration, separator layer filler concentration, separator layer filler type, and/or separator layer filler size. In some embodiments, the amount of electrolyte printed is controllable using different mesh sizes during a screen printing process. Excess electrolyte can be wiped away, or trial and error can be performed to select a desired or appropriate mesh size for the screen printing process after an initial estimate based on, for example, separator layer thickness and porosity, such that the printed electrolyte remaining in the printed battery is in the pores of the separator layer. A printed electrolyte may reduce, eliminate, or substantially eliminate an electrolyte dosimetry process, which can be used for filling a separator having no electrolyte with electrolyte. In some embodiments, a printed battery including an electrolyte layer which is printed onto a separator can facilitate improved ionic conductivity between electrodes of the printed battery, for example as compared to a battery having an electrolyte which is incorporated as part of an ink for the separator layer and is printed as part of the separator layer (e.g., having an improved ionic conductivity as compared to solid state electrolytes, such as electrolytes which are included in an ink for forming the separator layer, where the ink for the separator layer includes, for example, one or more ionic liquids, a material for providing structural strength for the separator layer, such as a polymeric material, and/or a solvent, and where the solvent is removed or substantially removed in the battery fabrication process).

In some embodiments, a separator sealing layer (e.g., the separator sealing layer as described with reference to Figure 7, the separator sealing layer as described with reference to Figure 8) is printed over a porous separator layer (e.g., the separator layer 73 of Figure 7, the separator layer 73 of Figure 8, the separator layer 90 of Figure 9). For example, the separator sealing layer can be printed subsequent to printing the electrolyte layer on the porous separator layer. A separator sealing layer may facilitate sealing of pores of the porous separator layer. For example, a separator sealing layer may advantageously facilitate sealing of pores of the porous separator layer prior to printing a second electrode layer, such as to provide improved retention of the printed electrolyte within the porous separator layer, and/or improved adhesion between the porous separator layer and the second electrode layer. In some embodiments, a separator sealing layer can reduce and/or prevent or substantially prevent seeping of one or more adjacent layers into the separator layer (e.g., seeping of a first electrode layer and/or a second electrode layer into the separator layer), for example due to pressure (e.g., pressure applied during a subsequent printing process). In some embodiments, the separator sealing layer can have a composition similar to that of an intermediate layer, such as the intermediate layer 3 shown in Figure 1. In some embodiments, the separator sealing layer may or may not include an electrolyte.

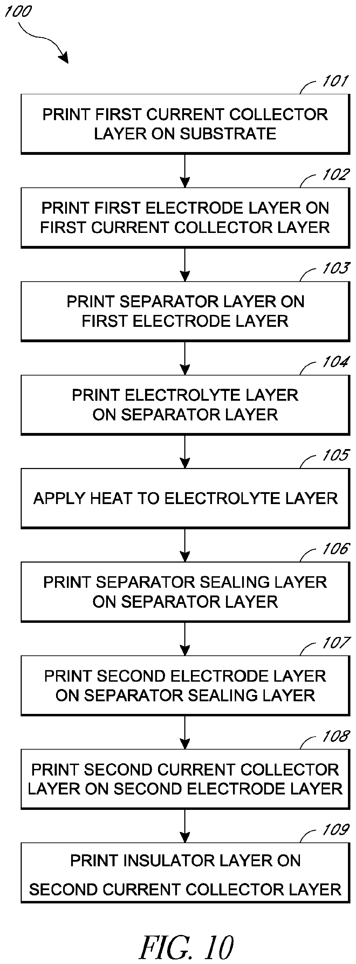

Figure 10 shows an embodiment of an example process for fabricating a printed battery (e.g., printed battery 70, and/or printed battery 80). In block 101, a first current collector layer can optionally be printed over and/or on a substrate. As described herein, a first current collector may be omitted if the substrate is or comprises conductive material. In block 102, a first electrode layer can be printed over and/or on the first current collector layer. In block 103, a separator layer can be printed over and/or on the first electrode layer. In block 104, an electrolyte can be printed over and/or on the separator layer. As described herein, the printed electrolyte can diffuse into the pores of the separator layer to provide a separator layer including a plurality of pores having the electrolyte (e.g., a plurality of pores filled or substantially filled with the electrolyte). In block 105, heat can be applied to the printed electrolyte layer, for example to facilitate diffusion of the printed electrolyte into the separator layer. The heating process may facilitate diffusion of the electrolyte into the pores of the separator layer by reducing viscosity of the printed electrolyte layer, for example reducing a duration in which all or substantially all of the printed electrolyte diffuses into the separator layer. In block 106, a separator sealing layer (e.g., an intermediate layer) can optionally be printed on the separator layer, for example subsequent to the heating process. In some embodiments, no or substantially no electrolyte remains on the separator layer when the subsequent layer is printed on the separator layer. A separator sealing layer may be printed over the separator layer (e.g., as described with respect to block 106) and/or under the separator layer (e.g., between block 102 and block 103). For example, separator sealing layers can be printed both prior to printing the separator layer and subsequent to printing the electrolyte on the separator layer (e.g., subsequent to the diffusion of all or substantially all of the printed electrolyte into the separator layer). In some embodiments, a separator sealing layer is printed subsequent to applying heat to the printed electrolyte. The separator sealing layer may facilitate reduction of seepage of the electrolyte into one or more layers adjacent to the separator layer and/or seepage of one or more layers adjacent to the separator layer into the separator layer, such as due to pressure applied to the printed layers during the battery fabrication process. In some embodiments, the printed battery includes no separator sealing layer above and/or below the separator. In block 107, a second electrode layer can be printed over and/or on the separator sealing layer. In block 108, a second current collector layer can be printed over and/or on the second electrode layer. In block 109, an insulator layer can be printed over and/or on the second current collector layer. In some embodiments, the insulator layer facilitates sealing of the printed battery. For example, the insulator layer may form an enclosure around the printed electrode layers and the printed separator layer.

It was unexpectedly discovered that applying a centrifugal force (e.g., centrifuging) to a solution including a salt and an ionic liquid can provide a solution having two or more distinct or substantially distinct phases, such as an upper phase and a lower phase. In some embodiments, an upper phase of the centrifuged solution is a liquid phase. In some embodiments, a lower phase of the centrifuged solution has an increased viscosity as compared to the upper phase (e.g., the lower phase can be a gel-like substance).

For example, a 0.7 mol/l (M) solution including zinc tetrafluoroborate in 1-ethyl-3-methylimidazolium tetrafluoroborate centrifuged at 10000 revolutions per minute (rpm) for 5 minutes (min) can provide a solution separated into two phases. An upper phase can be a transparent liquid having a concentration of about 0.6 M to about 0.7 M zinc tetrafluoroborate. A lower phase can be a gel having a concentration of about 0.7 M to about 0.8 M zinc tetrafluoroborate. In some embodiments, the lower phase can have an ionic conductivity similar to that of the upper phase. Other solution concentrations, speeds of centrifugation and/or durations of centrifugation may also be suitable. In some embodiments, the solution can be centrifuged at a speed of about 3,000 rpm to about 30,000 rpm, including about 3,000 rpm to about 15,000 rpm. In some embodiments, the solution can be centrifuged for a duration of about 30 seconds to about 15 minutes, including about 1 minute to about 10 minutes. For example, higher speeds and/or longer durations may be used for solutions with a higher initial concentration and/or specific gravity, and lower speeds and/or shorter durations may be used for solutions with a lower initial concentration and/or specific gravity. For another example, higher speeds can be used with lower durations, and lower speeds can be used with higher durations. The concentration of each phase can be measured by ion chromotography (e.g., commercially available from Metrohm USA Inc., of Riverview, Florida).

The separation of the Zn salt solution in ionic liquid into two phases is a previously unknown phenomena. An upper phase and a lower phase can be solutions of zinc salt in ionic liquid (e.g., a zinc salt dissolved in an ionic liquid to a molecular level). In some embodiments, the lower phase includes a molecular agglomerations of ionic liquid around zinc cations (e.g., at least partially due to a tendency of ionic liquids to form agglomeration). The upper phase may be relatively depleted of Zn ions as compared to the initial concentration of the electrolyte (e.g., prior to centrifuging). The lower phase may be relatively enriched with Zn ions as compared to the initial concentration of the electrolyte (e.g., prior to centrifuging).

The lower phase can have a consistency of a printable ink (e.g., more viscous than the ionic liquids and/or the non-centrifuged mixture of the zinc salts and ionic liquids).

In some embodiments, the upper phase can be used elsewhere in the battery fabrication process, for example as an additive to an ink for a Zn electrode.

According to the invention, styrene butadiene rubber is used as a binder material for an electrode of a printed battery, and, optionally, for a separator layer of a printed battery. This is a suspension of rubber, not a dissolved polymer in a solvent. For example, when electrodes and/or a separator layer are dried, the rubber may not solidify to form a continuous film. In some embodiments, an electrode including a suspension of rubber facilitates formation of pathways between active particles of the electrode (e.g., bridges between the active particles), for example providing more room for the electrolyte to contact the active particles. In some embodiments, a separator layer including a suspension of rubber facilitates ionic transport between electrodes of the printed battery, for example providing more room for mobility of the electrolyte.

In some embodiments, the styrene butadiene rubber may increase flexibility of the printed layers including the styrene butadiene rubber and/or the printed device as a whole.

In some embodiments, styrene butadiene rubber can be used in the fabrication of Zn/Mn02 batteries, such as in the fabrication of one or more electrodes of the batteries, and/or a separator layer of the batteries.

In some embodiments, a printed battery (e.g., printed battery 70 of Figure 7, printed battery 80 of Figure 8) includes one or more printed layers being printed from an ink having a water and/or water soluble solvent as the main solvent. In some embodiments, each layer of a printed battery can be printed from an ink having one or more water and/or water soluble solvents. For example, water, a water-like solvent (e.g., an alcohol), and/or water-soluble solvent (e.g., an alcohol) can be an ink carrier (e.g., a volatile component, a vaporizable element, and/or a viscosity modifier, of the ink) for the polymeric component of the ink used in printing the one or more printed layers. An ink for the one or more printed layers can be a colloidal suspension including small particles of the polymeric component in the water-soluble solvent. Drying of the colloidal suspension in forming the printed layer may form a non-continuous film, which may facilitate improved conductivity of the electrolyte, for example facilitating movement of the electrolyte through the film. In some embodiments, a battery (e.g., all layers of a printed battery) having water and/or water soluble solvents as the main solvent can facilitate ecologically friendly processes in the manufacturing of the battery, for example, as compared to polyvinylidene fluoride (PVDF) based batteries, facilitate fabrication of batteries having improved electrical performance (e.g., improved conductivity), and/or facilitate reduced costs of manufacturing. For example, PVDF based batteries can often include toxic solvents for dissolving the PVDF, and may use a significant amount of energy for the dissolution of PVDF in the toxic solvents. PVDF can be more expensive than the polymers described herein. In some embodiments, expensive PVDF is fully dissolved, using much energy, to form a true solution in the toxic solvents. A layer printed from a PVDF solution can dry as a continuous film, which may disadvantageously slow electrolyte travel through the film (e.g., because there are no discontinuities through which the electrolyte may travel), reducing conductivity of the continuous film and/or the printed battery made with the continuous film.

In some embodiments, water and/or water soluble solvents have lower boiling points than solvents used for PVDF, facilitating use of lower drying temperatures for the printed films and/or reduced time for drying the printed films.

### Example Compositions of Printed Battery Layers

An example composition of a first electrode layer according to the invention (e.g., referring again to Figures 7 and 8, the first electrode layer 72), such as a Zn electrode, by weight:
Styrene Butadiene Rubber (SBR) - 1.04%
Zn powder (particle size below 10 microns) - 97.2%
Electrolyte 1 mol/l (M) ZnBF₄ in C₂mimBF₄ - 1.76%

An example composition of a separator layer (e.g., separator layer 73), such as a separator layer including microspheres (e.g., glass spheres), by weight:
Polytetrafluoroethylene (PTFE) - 2.34% (Alternatively or in addition, styrene butadiene rubber (SBR) can be used. For example the separator layer can include 2.34% by weight of styrene butadiene rubber.)
Glass spheres (less than 20 microns in diameter) - 95.32%
PVA (polyvinyl alcohol) 133,000 molecular weight - 0.38%
PVA 6,000 molecular weight - 1.96%
The separator's pores can be filled with printed gel electrolyte (e.g., as described herein) having a concentration between about 0.7 M and about 0.8 M zinc tetrafluoroborate in 1-ethyl-3 -methylimidazolium tetrafluoroborate.

An example composition of a separator sealing layer (e.g., the separator sealing layer as described with reference to Figure 7, the separator sealing layer as described with reference to Figure 8), by weight:
Polyvinyl alcohol (PVA) 133,000 molecular weight - 6.86%
PVA 6,000 molecular weight - 35.73%
Electrolyte 0.6 mol/l (M) Zn(BF₄)₂ in C₂mimBF₄- 57.41%

An example composition of a second electrode layer (e.g., referring again to Figures 7 and 8, the second electrode layer 74) such as a MnO₂ electrode, by weight:
Styrene Butadiene Rubber - 4.3%
Manganese dioxide (MnO₂) - 77.3%
Multiwall carbon nanotubes (MWCNT) - 2.8% (it can be any mixture of MWCNTs and graphite or graphene)
Ionic liquid (1-ethyl-3-methylimidazolium tetrafluoroborate, C₂mimBF₄) - 15.6%

Current collectors (e.g., referring again to Figures 7 and 8, the first current collector 71 and the second current collector 75) can have a similar or same composition as other current collectors described herein (e.g., the current collectors 1, 6 of Figure 1). As described herein, in some embodiments, the first current collector 71 and the second current collector 75 can comprise or be made of a conductive nickel-containing film. Examples of embodiments of a nickel-containing film are provided in pages 21-23 and 25-33 of U.S. Provisional Patent Application No. 61/747,093, filed December 28, 2012.

An example composition for an insulator layer (e.g., referring again to Figures 7 and 8, the insulator layer 76), such as a polymer insulator layer, by weight:
Specialty copolymer based on vinylidene chloride and vinyl chloride monomers (e.g., Ixan® SGA-1, available from Solvay Specialty Polymers USA, LLC, of Alpharetta, Georgia) - 100%

### Example Compositions of Printable Ink Compositions (examples of successful ink compositions), Preparation Process, Properties and Curing Conditions

An example composition of an ink for forming a first electrode according to the invention (e.g., the electrode layer 72 of Figure 7 and/or Figure 8), such as a Zn electrode, by weight:
Styrene Butadiene Rubber (SBR) - 0.9%
Zn powder (particle size below 10 microns) - 83.9%
Water - 12.11%
N-butanol - 2%
Zinc tetrafluoroborate Zn(BF₄) - 0.14%
C₂mimBF₄ - 1.36%

An example procedure to prepare the ink for a printed electrode layer (e.g., the first electrode layer 72 in Figures 7 and 8) can include:
Combine a 15% SBR suspension in water such as a Modified Styrene Butadiene copolymer hydrophilic binder (e.g., PSBR-100 available from Targray, of Quebec, Canada), an upper phase of an electrolyte (see description of the electrolyte preparation herein), and water for achieving good viscosity. Preheat the combination to 40 °C and stir for 30 min using laboratory egg.

The ink for the electrode layer fabricated according to the example method can have a viscosity of about 8000 cP. An example curing profile for this composition is at a temperature of 100 °C, for between 3 and 5 minutes.

An example composition of an ink for forming a separator layer (e.g., the separator layer 72 of Figures 7 and 8), such as a separator layer including glass spheres, by weight:
Polytetrafluoroethylene (PTFE) - 1.43% (Alternatively or in addition, styrene butadiene rubber (SBR) can be used. For example 1.43% by weight of styrene butadiene rubber can be used.)
Glass spheres (less than 20 microns in diameter) - 58.3%
Polyvinyl alcohol (PVA) 133,000 molecular weight - 0.23%
PVA 6,000 molecular weight - 1.20%
Water - 36.83%
N-Butanol - 2%

An example procedure to prepare the ink for the printed separator layer 73 can include:
Prepare a PVA base: preheat water to 40 °C, add PVA 133K and PVA 6K and mix until dissolved using laboratory egg. Add 60% PTFE suspension in water (e.g., polytetrafluoroethylene preparation, 60 wt % dispersion in H₂O, available from Sigma-Aldrich, of Switzerland) and glass spheres to the PVA base. Mix for 10 minutes at room temperature using laboratory egg.

The ink for the separator layer fabricated according to the example method can have a viscosity of about 10000 cP. An example curing profile for this composition is at 100 °C, for between 3 and 5 minutes.

An example composition of an ink for the printed electrolyte, by weight:
0.7-0.8 mol/l viscous solution of zinc tetrafluoroborate in 1-ethyl-3-methylimidazolium tetrafluoroborate obtained after centrifuging (e.g., a lower phase of the centrifuged solution).

An example procedure to prepare the ink for the printed electrolyte can include:
Mixing the electrolyte with a magnetic stir bar for 2 hours at 40 °C, the electrolyte having the following composition, by weight:
   Ionic liquid (1-ethyl-3-methylimidazolium tetrafluoroborate, C₂mimBF₄) - 90.57% Zinc tetrafluoroborate (Zn(BF₄)₂) - 9.43%.
Centrifuge the mixture at 10,000 rpm for 5 minutes. Two phases appear: transparent upper phase and a gel-like phase (e.g., a lower phase). The lower phase can be used as or for the printable electrolyte.

The ink for the printed electrolyte fabricated using the example method can have a viscosity of about 5000 cP. An example curing profile for this composition is at a temperature of 100 °C, for between 3 and 5 minutes.

An example composition of an ink for a separator sealing layer, by weight:
Water - 81.19%
Polyvinyl alcohol (PVA) 133,000 molecular weight - 1.29%
PVA 6,000 molecular weight - 6.72%
Electrolyte 1 mol/l (M) Zn(BF₄)₂ in C₂mimBF₄ - 10.8% As described herein, the separator sealing layer can be with electrolyte or without electrolyte.

An example procedure to prepare the ink for the separator sealing layer can include:
Preheat water to 60 °C to 80 °C. Slowly pour PVA 133,000 molecular weight. Mix using magnetic bar. Higher temperatures can dissolve the PVA more quickly. Add PVA 6000 molecular weight when the PVA 133,000 is dissolved. Reduce heat to 60 °C and add electrolyte drop wise. Stir using laboratory egg. Cool down the mixture until gelled.

The ink for the separator sealing layer fabricated using the example method can have a viscosity of about 100 cP. An example curing profile for this composition is at a temperature of 130 °C, for between 5 and 7 minutes.

An example composition of an ink for forming a second electrode layer (e.g., the second electrode layer 74 of Figure 7 and/or Figure 8), such as a MnO₂ electrode, by weight:
Styrene Butadiene Rubber (SBR) - 2.3%
Manganese dioxide (MnO₂) - 41.1%
MWCNT (multiwall carbon nanotubes) - 1.48% (any mixture of MWCNTs and graphite or graphene may be appropriate)
Ionic liquid (1-ethyl-3-methylimidazolium tetrafluoroborate, C₂mimBF₄) - 8.29%
Water - 50.79%
Butanol - 2%

An example procedure to prepare the ink for the second electrode layer 74 can include:
Preparation of a MWCNTs paste in C₂mimBF₄ can be performed, for example, by two techniques:
A first technique can include taking 15% of MWCNTs and 85% of C₂mimBF₄, and grinding the mixture in a mortar and pestle in glove box for 5 min, then grind in automated mortar and pestle for 1 hour (e.g., such a technique can be suitable for small amounts). Grinding of the mixture can advantageously facilitate untangling of agglomerated carbon nanotubes, for example to facilitate solvation of the carbon nanotubes in the ionic liquid. Grinding of the mixture can facilitate untangling of agglomerated carbon nanotubes such that the carbon nanotubes are evenly or substantially evenly dispersed within the ionic liquid.
A second technique can include taking 15% of MWCNTs and 85% of C₂mimBF₄, and mixing for one day in a jar mill with zirconia grinding media (diameter of zirconia beads is 0.5-2 cm) (e.g., such a technique can be suitable for larger amounts). This forms a bucky gel in which the carbon nanotubes are evenly or substantially evenly dispersed in the ionic liquid C₂mimBF₄, for example such that the carbon nanotubes do not agglomerate. As described herein, grinding of the mixture comprising the carbon nanotubes and the ionic liquid can advantageously facilitate untangling of agglomerated carbon nanotubes and/or solvation of the carbon nanotubes in the ionic liquid.
Take SBR and mix with MWCNT paste under sonication and 50 °C for 30 min. Graphite and/ or graphene can be optionally added to MnO₂ powder and mixed at 70 °C for 90 min using laboratory egg.

The ink for the second electrode layer fabricated using the example method can have a viscosity of about 9000 cP. An example curing profile for this composition is at a temperature of 100 °C, for between 3 and 5 minutes.

Referring again to Figures 7 and 8, one or both of the first current collector 71 and the second current collector 75 can have a similar or same composition as other current collectors described herein (e.g., the current collectors 1 and 6 of Figure 1), and can be prepared according to methods as described herein. As described herein, in some embodiments, the first current collector 71 and the second current collector 75 can include nickel. For example, the first current collector 71 and the second current collector 75 can be made of a nickel-containing film, example compositions of and methods of fabricating a nickel-containing film are provided in pages 21-23 and 25-33 of U.S. Provisional Patent Application No. 61/747,093, filed December 28, 2012.

An example composition of an ink for forming an insulator layer (e.g., the insulator layer 76 of Figure 7 and/or Figure 8), by weight:
Specialty copolymer based on vinylidene chloride and vinyl chloride monomers (e.g., Ixan® SGA-1, available from Solvay Specialty Polymers USA, LLC, of Alpharetta, Georgia) - 10%
N-Butanol solvent - 90%

An example procedure to prepare the ink for the insulator layer can include:
Heat butanol to 60 °C and progressively add the copolymer. Mix for 30 min at 60 °C using laboratory egg.

The ink for the insulator layer fabricated using the example method can have a curing profile at a temperature of 130 °C, for between 3 and 5 minutes.

### Examples of Physical Parameters and Electrical Performance of a Printed Battery

Example thicknesses of the printed layers of a printed battery (e.g., the printed battery 70 of Figure 7 or the main lateral portions of the printed battery 80 of Figure 8) can include:
1. First current collector: 5-25 micrometers (µm) or 0 µm (e.g., if the substrate comprises or is electrically conductive material)
2. Zinc electrode: 20-70 micrometers (µm), depending on substrate and absence/presence of the first current collector
3. Glass sphere separator: 10-30 micrometers (µm)
4. Printed electrolyte is completely adsorbed in the glass separator pores.
5. Separator sealing layer: less than 1 micrometer (µm)
6. MnO₂ electrode: 20-60 micrometers (µm)
7. Second current collector: 5-25 micrometers (µm)
8. Protective layer: around 10 micrometers (µm)
Overall thickness of the printed battery: 70-220 micrometers (µm)

Substrates can have thickness 10-200 micrometers (µm) making the total thickness of the device about 400 micrometers (µm). On thin substrates (e.g., substrates having a thickness of about 30 to about 60 micrometers (µm)) the overall battery thickness can be as thin as about 130 micrometers (µm).

The printed batteries can be printed in any suitable shape. For example, referring again to Figure 8, some portions of the 73 overlap the layers 71 and/or 72. For another example, although shown as an example cross-section or side elevational view, a top plan view can include shapes such as quadrilaterals, other polygons, round shapes, combinations thereof, etc.

Using suspensions of polymers (e.g., according to the invention, Styrene Butadiene Rubber; and, optionally, PTFE) rather than polymers dissolved in solvents in most of the layers, and having a rubber (according to the invention, Styrene Butadiene Rubber) as a binder material of the electrodes, can facilitate improved flexibility of the battery.

### Details of Battery Performance

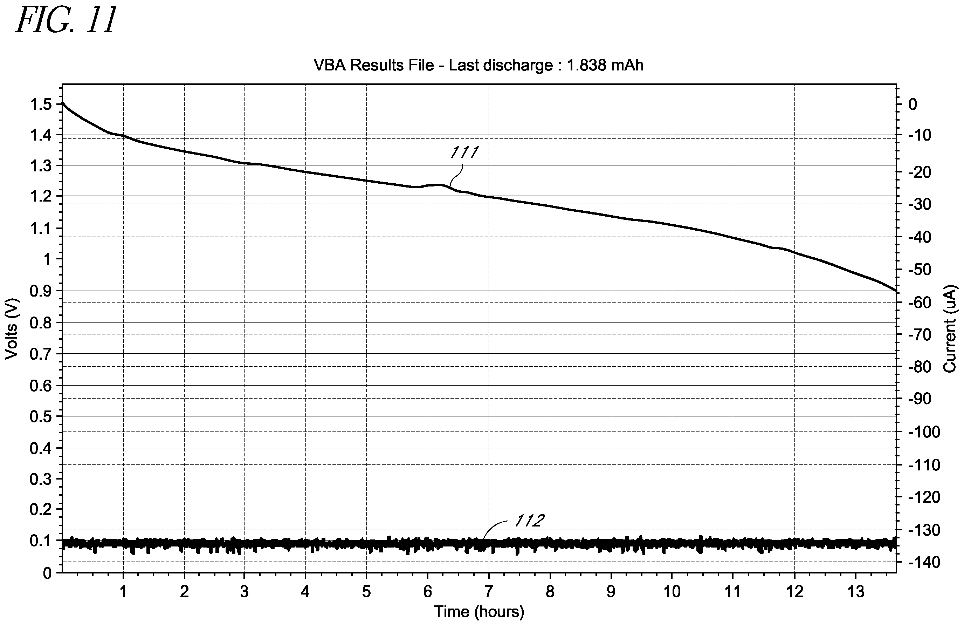

Graph 111 of Figure 11, with reference to the y-axis labeled with voltage in Volts (V), is a graph of battery potential versus time for discharge of an example printed battery including the layers described with respect to Figures 7 and 8 and having a rectangular shape and dimensions of 1 centimeter (cm) by 2 cm. Graph 112 of Figure 11, with references to the y-axis labeled with of current in microamperes (µA), shows that the printed battery was discharged at constant current 0.137 milliAmperes (mA) (or 0.068 mA/cm²) (e.g., a constant current discharge curve). Measurements were performed on Vencon UBA5 Battery Analyzer (e.g., available from Vencon Technologies, Inc., of Ontario, Canada). The printed battery was discharged with the current of 0.137 (mA) and lasted 14 hours. The cut-off voltage for the calculations was 900 millivolts (mV). The capacity of the printed Zn/MnO₂ battery is about 1.838 milliampere-hour (mAh). The open circuit potential of the printed battery is 1.56 V. Below are the readings of a Vencon UBA5 Battery Analyzer for a printed battery having the above-listed physical characteristics (e.g., a printed zinc manganese dioxide, ZnMnO₂, battery):
Number of cells: 1
Rated capacity: 1.000 milliampere-hour (mAh)
Open circuit Voltage: 1.5566 Volts (V)
Relative Humidity: 22%
Curing: 290 °F for 8 min
Capacity: 1.838 milliampere-hour (mAh) (183.8% rated)

### Summary of electrical performance of the printed battery:

ZnMnO₂ discharge:
Pass, Capacity=1.838 milliampere-hour (mAh) (183.8% rated), IPV=1.489 Volts (V), MPV=0.914 Volts (V), EPV=0.900 Volts (V), MPI=134.2 microamperes (µA).
Duration (hour:minute:second): 13:40:29
Load current: 137.1 microamperes (µA)
Cut-off voltage: 900 mV
Battery discharged capacity: 1.838 milliampere-hour (mAh)
Battery initial-point voltage: 1.489 Volts (V)
Battery mid-point voltage: 914 millivolts (mV)
Battery end-point voltage: 900 millivolts (mV)
Battery midpoint current: 134.2 microamperes (µA)
Exit condition: Battery cut-off voltage reached.

### Printing

The final composition of the layers may be formed after printing a corresponding ink and drying (curing) the layer at least a certain temperature for at least a certain time.

Inks generally have all the components of the corresponding layers plus one or more water soluble, and/or organic solvents. The solvents can be used to dissolve polymers (e.g., acting as solvents) and/or to create a suitable viscosity for printing of the inks (e.g., acting as viscosity modifiers) that evaporate during the drying process.

The printed zinc manganese dioxide battery can be printed on any flexible or rigid substrate that is conductive or non-conductive. Choice of water soluble and/or organic solvents often depends on the ability of the solvent to wet substrates (e.g., acting as wetting agents). In some embodiments, a printing ink comprises a second solvent to provide increased wettability of the substrate.

In some embodiments, the printing ink is suitable for a screen printing process. The printing ink may also be suitable for other printing processes.

### LIST OF EXAMPLE ALTERNATIVE COMPONENTS AND TECHNIQUES

### Polymers

Suitable polymers for one or more layers of the printed battery 10, or a printed battery 70, include, but are not limited to: (or equivalently, polymeric precursors or polymerizable precursors) such as polyvinyl pyrrolidone (PVP), polyvinyl alcohol (PVA), polyvinylidene fluoride, polyvynylidene fluoride-trifluoroethylene, polytetrafluoroethylene (PTFE), polydimethylsiloxane, polyethelene, polypropylene, polyethylene oxide, polypropylene oxide, polyethylene glycolhexafluoropropylene, polyethylene terefphtalatpolyacrylonitryle, polyvinyl butyral, polyvinylcaprolactam, polyvinyl chloride; polyimide polymers and copolymers (including aliphatic, aromatic and semi-aromatic polyimides), polyamides, polyacrylamide, acrylate and (meth)acrylate polymers and copolymers such as polymethylmethacrylate, polyacrylonitrile, acrylonitrile butadiene styrene, allylmethacrylate, polystyrene, polybutadiene, polybutylene terephthalate, polycarbonate, polychloroprene, polyethersulfone, nylon, styrene-acrylonitrile resin; polyethylene glycols, clays such as hectorite clays, garamite clays, organomodified clays; saccharides and polysaccharides such as guar gum, xanthan gum, starch, butyl rubber, agarose, pectin; celluloses and modified celluloses such as hydroxyl methylcellulose, methylcellulose, ethyl cellulose, propyl methylcellulose, methoxy cellulose, methoxy methylcellulose, methoxy propyl methylcellulose, hydroxy propyl methylcellulose, carboxy methylcellulose, hydroxy ethylcellulose, ethyl hydroxyl ethylcellulose, cellulose ether, cellulose ethyl ether, and/or chitosan.

In some embodiments, a suitable polymer may be electrostable under a working voltage of a battery. A Zn/MnO₂ battery has relatively low voltage, so many polymers may be suitable. For example, fluorinated polymers may be suitable due to their chemical, thermo, and electrochemical stability.

The insulator layer 7, or the insulator layer 76, may include a polymer that does not allow penetration by oxygen and/or water into the battery. A variety of PVDF or polyolefins can be used as barrier layer polymers for both water and oxygen. A combination of an oxygen barrier polymer and a moisture barrier polymer from the list above is also possible.

### Solvents

Suitable solvents used in preparing one or more inks for fabricating a printed battery 10, a printed battery 70, a printed battery 80, etc., include, but are not limited to: water, alcohols such as methanol, ethanol, N-propanol (including 1-propanol, 2-propanol (isopropanol or IPA), 1-methoxy-2-propanol), butanol (including 1-butanol, 2-butanol (isobutanol)), pentanol (including 1- pentanol, 2- pentanol, 3- pentanol), hexanol (including 1-hexanol, 2-hexanol, 3- hexanol), octanol, N-octanol (including 1-octanol, 2-octanol, 3-octanol), tetrahydrofurfuryl alcohol (THFA), cyclohexanol, cyclopentanol, terpineol; lactones such as butyl lactone; ethers such as methyl ethyl ether, diethyl ether, ethyl propyl ether, and polyethers; ketones, including diketones and cyclic ketones, such as cyclohexanone, cyclopentanone, cycloheptanone, cyclooctanone, acetone, benzophenone, acetylacetone, acetophenone, cyclopropanone, isophorone, methyl ethyl ketone; esters such ethyl acetate, dimethyl adipate, proplyene glycol monomethyl ether acetate, dimethyl glutarate, dimethyl succinate, glycerin acetate, carboxylates; carbonates such as propylene carbonate; polyols (or liquid polyols), glycerols and other polymeric polyols or glycols such as glycerin, diol, triol, tetraol, pentaol, ethylene glycols, diethylene glycols, polyethylene glycols, propylene glycols, dipropylene glycols, glycol ethers, glycol ether acetates 1,4- butanediol, 1,2-butanediol, 2,3-butanediol, 1,3-propanediol, 1,4-butanediol, 1,5- pentanediol, 1,8-octanediol, 1,2-propanediol, 1,3-butanediol, 1,2-pentanediol, etohexadiol, p-menthane-3,8-diol, 2-methyl-2,4-pentanediol; tetramethyl urea, n-methylpyrrolidone, acetonitrile, tetrahydrofuran (THF), dimethyl formamide (DMF), N-methyl formamide (NMF), dimethyl sulfoxide (DMSO); thionyl chloride; and/or sulfuryl chloride.

Higher boiling point solvents are generally preferable for printing. A slow evaporation rate can reduce solvent loss during ink mixing and printing, as can influence the shelf life of an ink comprising the solvent.

### Ionic Liquids

Ionic liquids (ILs) are generally organic molten salts which consist only of ions and are liquid at temperatures below 100 °C. Every ionic liquid has a cation and anion. Suitable ionic liquids can be any combination from the list of cations and the list of anions below. For example, an IL described herein is C₂mimBF₄, which is a combination of the first cation and the first anion listed below.

Suitable cations are selected from 1-ethyl-3-methylimidazolium, butyltrimethylammonium, 1-butyl-3-methylimidazolium, 1-methyl-3-propylimidazolium, 1-hexyl-3-methylimidazolium, choline, ethylammonium, tributylmethylphosphonium, tributyl(tetradecyl)phosphonium, trihexyl(tetradecyl)phosphonium, 1-ethyl-2,3-methylimidazolium, 1-butyl-1-methylpiperidinium, diethylmethylsulfonium, 1-methyl-3-propylimidazolium, 1-methyl-1-propylpiperidinium, 1-butyl-2-methylpyridinium, 1-butyl-4-methylpyridinium, 1-butyl-1-methylpyrrolidinium, diethylmethylsulfonium, and the like.

Suitable anions are selected from tetrafluoroborate, tris(pentafluoroethyl)trifluorophosphate, hexafluorophosphate, ethyl sulfate, dimethyl phosphate, trifluoromethanesulfonate, methansulfonate, triflate, tricyanomethanide, dibutylphosphate, bis(trifluoromethylsulfonyl)imide, bis-2,4,4-(trimethylpentyl) phosphinate, iodide, chloride, bromide, nitrate, and the like.

### Zinc Salts

Suitable zinc salts may include, but are not limited to: zinc chloride, zinc bis(trifluoromethanesulfonyl)imide, zinc sulfate, zinc nitrate, and/or zinc carbonate, and the like.

Other examples of suitable zinc salts may include combinations of zinc cation with organic and inorganic anions. In some embodiments, suitable zinc salts have desired solubility in the ionic liquid.

### Microspheres

Suitable solid microspheres for the separator layer 4, the separator layer 73, etc., may be hollow or dense, and may be spherical or substantially spherical particles comprising non-conductive materials like glass, alumina, silica, polystyrene, and/or melamine. The solid microsphere particles size may have a diameter from about 0.5 µm to about30 µm.

### Substrates

Substrates can be conductive and/or non-conductive. Example substrates include, but are not limited to: graphite paper, graphene paper, polyester film, polyimide film, Al foil, copper (Cu) foil, stainless steel (SS) foil, carbon foam, polycarbonate film, paper, coated paper, plastic coated paper, fiber paper, and/or cardboard, and the like.

### Printing Techniques

"Printing" includes any and all printing, for example, coating, rolling, spraying, layering, spin coating, laminating and/or affixing processes, for example, screen printing, inkjet printing, electro-optical printing, electroink printing, photoresist and other resist printing, thermal printing, laser jet printing, magnetic printing, pad printing, flexographic printing, hybrid offset lithography, Gravure and other intaglio printing, die slot deposition, and the like.

### Ink Preparation Techniques

All kinds of ink mixing techniques are possible, including, but not limited to: mixing with stir bar, mixing with magnetic stirrer, vortexing (Vortex machine), shaking (using shakers), mixing by rotation, sonication, mortar and pestle, and the like.

### Curing Conditions

Suitable temperatures for curing an ink used in printing one or more of the battery layers can have a value in a wide temperature range depending on solvents used, for example from about 70 °C to about 300 °C. Drying time can vary from about 20 seconds to about 1 hour.

A suitable atmosphere for curing an ink used in printing one or more of the battery layers can be ambient, inert, or vacuum.

## Claims

1. A printed electrode (2, 5) for a printed energy storage device (10), the electrode (2, 5) comprising:
an electrolyte (3) comprising an ionic liquid and a zinc salt; and
styrene butadiene rubber, which is printed as a suspension,
wherein the ionic liquid (3) includes a cation selected from the group consisting of 1-ethyl-3- methylimidazolium, butyltrimethylammonium, 1-butyl-3-methylimidazolium, 1-methyl-3- propylimidazolium, 1-hexyl-3-methylimidazolium, choline, ethylammonium, tributylmethylphosphonium, tributyl(tetradecyl)phosphonium, trihexyl(tetradecyl)phosphonium, 1- ethyl-2,3-methylimidazolium, 1-butyl-1-methylpiperidinium, diethylmethylsulfonium, 1-methyl-3- propylimidazolium, 1-methyl-1-propylpiperidinium, 1-butyl-2-methylpyridinium, 1-butyl-4- methylpyridinium, 1-butyl-1-methylpyrrolidinium, and diethylmethylsulfonium,
wherein the ionic liquid (3) includes an anion selected from the group consisting of tetrafluoroborate, tris(pentafluoroethyl)trifluorophosphate, trifluoromethanesulfonate, hexafluorophosphate, tetrafluoroborate, ethyl sulfate, dimethyl phosphate, methansulfonate, triflate, tricyanomethanide, dibutylphosphate, bis(trifluoromethylsulfonyl)imide, bis-2,4,4-(trimethylpentyl) phosphinate, iodide, chloride, bromide, and nitrate,
wherein an anion of the zinc salt is the same as the anion of the ionic liquid.

2. The printed electrode (2, 5) of claim 1, further comprising carbon nanotubes.

3. The printed electrode (2, 5) of claim 2, wherein at least some of the carbon nanotubes comprise single-walled carbon nanotubes.

4. The printed electrode (2, 5) of claim 2 or 3, wherein at least some of the carbon nanotubes comprise multi-walled carbon nanotubes.

5. The printed electrode (5) of any one of claims 1 to 4, further comprising at least one of zinc and a conductive carbon, wherein the printed electrode is an anode (5) for a zinc manganese dioxide battery (10).

6. The printed electrode (2) of any one of claims 1 to 4, further comprising at least one of manganese dioxide and a conductive carbon, wherein the printed electrode is a cathode (2) for a zinc manganese dioxide battery (10).

7. The printed electrode (2, 5) of any one of claims 1 to 6, wherein the ionic liquid (3) comprises 1-ethyl-3- methylimidazolium tetrafluoroborate (C₂mimBF₄).

8. A printed energy storage device (10), comprising:
the printed electrode (2, 5) of any one of claims 1 to 7; and
a separator layer (4).

9. The printed energy storage device (10) of claim 8, wherein the separator layer (4) comprises a plurality of pores (93) and a liquid electrolyte in the plurality of pores (93).

10. The printed energy storage device (10) of claim 9, wherein the liquid electrolyte comprises the ionic liquid.

11. The printed energy storage device (10) of any one of claims 8 to 10, wherein the separator (4) comprises at least one of polytetrafluoroethylene (PTFE) and styrene butadiene rubber (SBR).

12. The printed energy storage device (10) of any one of claims 8 to 11, wherein the separator (4) comprises microspheres (91).

13. The printed electrode (2, 5) of any one of claims 1 to 7, further comprising at least one of graphite and graphene.

14. The printed electrode (2, 5) of any one of claims 2 to 4, wherein the carbon nanotubes are ground.

15. The printed electrode (2, 5) of any one of claims 2 to 4 and 14, wherein the carbon nanotubes and the ionic liquid (3) form a homogeneous mixture.

## Patentansprüche

1. Gedruckte Elektrode (2, 5) für eine gedruckte Energiespeichervorrichtung (10), wobei die Elektrode (2, 5) umfasst:
einen Elektrolyten (3), umfassend eine ionische Flüssigkeit und ein Zinksalz; und
Styrol-Butadien-Kautschuk, der als Aufhängung gedruckt ist,
wobei die ionische Flüssigkeit (3) ein Kation beinhaltet, ausgewählt aus der Gruppe bestehend aus 1-Ethyl-3-methylimidazolium, Butyltrimethylammonium, 1-Butyl-3-methylimidazolium, 1-Methyl-3-propylimidazolium, 1-Hexyl-3-methylimidazolium, Cholin, Ethylammonium, Tributylmethylphosphonium, Tributyl(tetradecyl)phosphonium, Trihexyl(tetradecyl)phosphonium, 1-Ethyl-2,3-methylimidazolium, 1-Butyl-1-methylpiperidinium, Diethylmethylsulfonium, 1-Methyl-3-propylimidazolium, 1-Methyl-1-propylpiperidinium, 1-Butyl-2-methylpyridinium, 1-Butyl-4-methylpyridinium, 1-Butyl-1-methylpyrrolidinium und Diethylmethylsulfonium,
wobei die ionische Flüssigkeit (3) ein Anion beinhaltet, ausgewählt aus der Gruppe bestehend aus Tetrafluoroborat, Tris(pentafluorethyl)trifluorophosphat, Trifluormethansulfonat, Hexafluorophosphat, Tetrafluoroborat, Ethylsulfat, Dimethylphosphat, Methansulfonat, Triflat, Tricyanomethanid, Dibutylphosphat, Bis(trifluormethylsulfonyl)imid, Bis-2,4,4-(trimethylpentyl)phosphinat, Jodid, Chlorid, Bromid und Nitrat,
wobei ein Anion des Zinksalzes das gleiche ist wie das Anion der ionischen Flüssigkeit.

2. Gedruckte Elektrode (2, 5) nach Anspruch 1, außerdem umfassend Kohlenstoff-Nanoröhrchen.

3. Gedruckte Elektrode (2, 5) nach Anspruch 2, wobei mindestens einige der Kohlenstoff-Nanoröhrchen einwandige Kohlenstoff-Nanoröhrchen umfassen.

4. Gedruckte Elektrode (2, 5) nach Anspruch 2 oder 3, wobei mindestens einige der Kohlenstoff-Nanoröhrchen mehrwandige Kohlenstoff-Nanoröhrchen umfassen.

5. Gedruckte Elektrode (5) nach einem der Ansprüche 1 bis 4, die außerdem mindestens eines von Zink und einem leitenden Kohlenstoff umfasst, wobei die gedruckte Elektrode eine Anode (5) für eine Zink-Mangandioxid-Batterie (10) ist.

6. Gedruckte Elektrode (2) nach einem der Ansprüche 1 bis 4, außerdem umfassend mindestens eines von Mangandioxid und einem leitfähigen Kohlenstoff, wobei die gedruckte Elektrode eine Kathode (2) für eine Zink-Mangandioxid-Batterie (10) ist.

7. Gedruckte Elektrode (2, 5) nach einem der Ansprüche 1 bis 6, wobei die ionische Flüssigkeit (3) 1-Ethyl-3-methylimidazoliumtetrafluoroborat (C2mimBF4) umfasst.

8. Gedruckte Energiespeichervorrichtung (10), umfassend:
die gedruckte Elektrode (2, 5) aus einer der Ansprüche 1 bis 7; und
eine Separatorschicht (4).

9. Gedruckte Energiespeichervorrichtung (10) nach Anspruch 8, wobei die Separatorschicht (4) mehrere Poren (93) und einen flüssigen Elektrolyten in den mehreren Poren (93) umfasst.

10. Gedruckte Energiespeichervorrichtung (10) nach Anspruch 9, wobei der flüssige Elektrolyt die ionische Flüssigkeit umfasst.

11. Gedruckte Energiespeichervorrichtung (10) nach einem der Ansprüche 8 bis 10, wobei der Separator (4) mindestens eines von Polytetrafluorethylen (**PTFE**) und Styrol-Butadien-Kautschuk (SBR) umfasst.

12. Gedruckte Energiespeichervorrichtung (10) nach einem der Ansprüche 8 bis 11, wobei der Separator (4) Mikrokugeln (91) umfasst.

13. Gedruckte Elektrode (2, 5) nach einem der Ansprüche 1 bis 7, außerdem umfassend mindestens eines aus Graphit und Graphen.

14. Gedruckte Elektrode (2, 5) nach einem der Ansprüche 2 bis 4, wobei die Kohlenstoff-Nanoröhrchen gemahlen sind.

15. Gedruckte Elektrode (2, 5) nach einem der Ansprüche 2 bis 4 und 14, wobei die Kohlenstoff-Nanoröhrchen und die ionische Flüssigkeit (3) eine homogene Mischung bilden.

## Revendications

1. Électrode imprimée (2,5) pour un dispositif imprimé de stockage d'énergie (10), l'électrode (2,5) comprenant :
un électrolyte (3) comprenant un liquide ionique et un sel de zinc ; et
du caoutchouc styrène-butadiène, qui est imprimé en tant que suspension,
dans laquelle le liquide ionique (3) comprend un cation choisi dans le groupe consistant en 1-éthyl-3-méthylimidazolium, butyltriméthylammonium, 1-butyl-3-méthylimidazolium, 1-méthyl-3-propylimidazolium, 1-hexyl-3-méthylimidazolium, choline, éthylammonium, tributylméthylphosphonium, tributyl(tétradécyl)phosphonium, trihexyl(tétradécyl)phosphonium, 1-éthyl-2,3-méthylimidazolium, 1-butyl-1-méthylpipéridinium, diéthylméthylsulfonium, 1-méthyl-3-propylimidazolium, 1-méthyl-1-propylpipéridinium, 1-butyl-2-méthylpyridinium, 1-butyl-4-méthylpyridinium, 1-butyl-1-méthylpyrrolidinium et diéthylméthylsulfonium,
dans laquelle le liquide ionique (3) comprend un anion choisi dans le groupe consistant en tétrafluoroborate, tris(pentafluoroéthyl)trifluorophosphate, trifluorométhanesulfonate, hexafluorophosphate, tétrafluoroborate, éthyl sulfate, diméthyl phosphate, méthanesulfonate, triflate, tricyanométhanide, dibutylphosphate, bis (trifluorométhylsulfonyle)imide, bis-2,4,4-(triméthylpentyl)phosphinate, iodure, chlorure, bromure et nitrate,
dans laquelle un anion du sel de zinc est le même que l'anion du liquide ionique.

2. Électrode imprimée (2, 5) selon la revendication 1, comprenant en outre des nanotubes de carbone.

3. Électrode imprimée (2, 5) selon la revendication 2, dans laquelle au moins une partie des nanotubes de carbone comprend des nanotubes de carbone à simple paroi.

4. Électrode imprimée (2, 5) selon l'une des revendications 2 ou 3, dans laquelle au moins une partie des nanotubes de carbone comprend des nanotubes de carbone à parois multiples.

5. Électrode imprimée (5) selon l'une quelconque des revendications 1 à 4, comprenant en outre au moins l'un du zinc et d'un carbone conducteur, l'électrode imprimée étant une anode (5) pour une pile zinc - dioxyde de manganèse (10) .

6. Électrode imprimée (2) selon l'une quelconque des revendications 1 à 4, comprenant en outre au moins l'un du dioxyde de manganèse et d'un carbone conducteur, l'électrode imprimée étant une cathode (2) pour une pile zinc - dioxyde de manganèse (10).

7. Électrode imprimée (2, 5) selon l'une quelconque des revendications 1 à 6, dans lequel le liquide ionique (3) comprend du tétrafluoroborate de 1-éthyl-3-méthylimidazolium (C₂mimBF₄).

8. Dispositif imprimé de stockage d'énergie (10), comprenant :
l'électrode imprimée (2, 5) selon l'une quelconque des revendications 1 à 7 ; et
une couche de séparation (4).

9. Dispositif imprimé de stockage d'énergie (10) selon la revendication 8, dans lequel la couche de séparation (4) comprend une pluralité de pores (93) et un électrolyte liquide dans la pluralité de pores (93).

10. Dispositif imprimé de stockage d'énergie (10) selon la revendication 9, dans lequel l'électrolyte liquide comprend le liquide ionique.

11. Dispositif imprimé de stockage d'énergie (10) selon l'une quelconque des revendications 8 à 10, dans lequel le séparateur (4) comprend au moins l'un du polytétrafluoroéthylène (PTFE) et du caoutchouc styrène-butadiène (SBR).

12. Dispositif imprimé de stockage d'énergie (10) selon l'une quelconque des revendications 8 à 11, dans lequel le séparateur (4) comprend des microsphères (91).

13. Électrode imprimée (2, 5) selon l'une quelconque des revendications 1 à 7, comprenant en outre au moins l'un du graphite et du graphène.

14. Électrode imprimée (2, 5) selon l'une quelconque des revendications 2 à 4, dans lequel les nanotubes de carbone sont broyés.

15. Électrode imprimée (2, 5) selon l'une quelconque des revendications 2 à 4 et 14, dans lequel les nanotubes de carbone et le liquide ionique (3) forment un mélange homogène.
